# EUROPEAN PATENT SPECIFICATION

(11) **EP 4 447 812 B1**
(45) Date of publication and mention of the grant of the patent: **03.09.2025**
(21) Application number: 22835707.5
(22) Date of filing: 12.12.2022
(51) Int. Cl.: A61B 6/08, A61B 90/00, G16H 20/40, G16H 40/63, A61B 34/20, A61N 5/10, A61B 90/50, A61B 6/00

(54) **COMPUTER-IMPLEMENTED METHOD FOR USE IN ALIGNING PIECES OF EQUIPMENT OF A MEDICAL SYSTEM**
COMPUTERIMPLEMENTIERTES VERFAHREN ZUR VERWENDUNG BEI DER AUSRICHTUNG VON AUSRÜSTUNGSTEILEN EINES MEDIZINISCHEN SYSTEMS
PROCÉDÉ MIS EN OEUVRE PAR ORDINATEUR DESTINÉ À ÊTRE UTILISÉ POUR ALIGNER DES ÉQUIPEMENTS D'UN SYSTÈME MÉDICAL

(30) Priority: 15.12.2021 WO PCT/EP2021/085965
(43) Date of publication of application: 23.10.2024
(73) Proprietor: Brainlab AG, 81829 München (DE)
(72) Inventor: KAISER, Hagen, 81829 München (DE); BERLINGER, Kajetan, 81829 München (DE)
(74) Representative: Maiwald GmbH
(86) International application number: PCT/EP2022/085432
(87) International publication number: WO 2023/110766

(56) References cited:
- US-A1- 2007 170 373
- US-A1- 2008 130 837
- US-A1- 2011 013 752

## Description

### FIELD OF THE INVENTION

The present invention relates to a computer-implemented method for use in aligning pieces of equipment of a medical system, a medical system, a computer program, a program storage medium, a computer, a signal wave, and a data stream representative of the program.

### TECHNICAL BACKGROUND

In general, alignment of pieces of equipment of a medical system, for example alignment with respect to a machine isocenter, is a cumbersome and time-consuming process. It often requires iterative adjustment, for example based on user intuition or experience, and repeated image taking to check the alignment after each adjustment. In case of radiation systems, an user must repeatedly leave the room due to the radiation, e.g., when taking X-ray images. The user may be the operator of the medical system.

As a consequence of the process being cumbersome and time-consuming, there is a risk that users will perform alignment less frequently than would be BEST for high-precision applications. That is, during operation of the system, gradual misalignment will usually occur that needs to be counteracted by performing alignment of the pieces of equipment. Otherwise, accuracy deteriorates over time.

Similarly, determining an isocenter of the machine may not be performed as frequently as would be required for high-precision applications, as it also usually relies on cumbersome iterative processes.

Thus, overall, alignment of the pieces of equipment is time consuming and inefficient.

The present invention has the object of providing a method allowing for an improved alignment of pieces of equipment of a medical system, particularly, a more efficient alignment.

The present invention can be used, e.g., in connection with medical systems like a system for image-guided radiotherapy such as VERO^{®} and ExacTrac^{®}, both products of Brainlab AG.

Aspects of the present invention, examples and exemplary steps and their embodiments are disclosed in the following. Different exemplary features of the invention can be combined in accordance with the invention wherever technically expedient and feasible.

US2011013752A1 uses markers in an X-ray image to determine if an imaging surface of a detector is inclined relative to the central ray of an X-ray beam.

### EXEMPLARY SHORT DESCRIPTION OF THE INVENTION

In the following, a short description of the specific features of the present invention is given which shall not be understood to limit the invention only to the features or a combination of the features described in this section.

The present invention provides a computer-implemented method for use in aligning pieces of equipment of a medical system, a medical system, a computer program, a program storage medium, a computer, a signal wave, and a data stream representative of the program according to the independent claims. Preferred embodiments are laid down in the dependent claims.

Specifically, the present disclosure provides a computer-implemented method for use in aligning pieces of equipment of a medical system, comprising determining, for a piece of equipment of a medical system, a viewing direction of the piece of equipment, the viewing direction being a normal to a predetermined portion of the surface of the piece of equipment, determining whether the viewing direction is within a predetermined interval around a target viewing direction of the piece of equipment, and upon determining that the viewing direction is outside of the predetermined interval around the target viewing direction of the piece of equipment, initiating an adjusting of the alignment of the piece of equipment.

### GENERAL DESCRIPTION OF THE INVENTION

In this section, a description of the general features of the present invention is given for example outlining possible embodiments of the invention.

The present disclosure provides a computer-implemented method for use in aligning pieces of equipment of a medical system. The method comprises determining, for a piece of equipment of a medical system, a viewing direction of the piece of equipment, the viewing direction being a normal to a predetermined portion of the surface of the piece of equipment. The method further comprises determining whether the viewing direction is within a predetermined interval around a target viewing direction of the piece of equipment, and, upon determining that the viewing direction is outside of the predetermined interval around the target viewing direction of the piece of equipment, initiating an adjusting of the alignment of the piece of equipment.

In other words, the present disclosure provides a method that uses normals of surfaces of pieces of equipment to determine whether their alignment requires correction and initiates the correction. Thus, a timely adjustment of alignment is enabled.

A viewing direction outside of the predetermined interval around the target viewing direction of the piece of equipment may indicate a misalignment of the piece of equipment.

An advantage of the method of the present disclosure is that it allows for improved, particularly more efficient, alignment of pieces of equipment.

For example, as detecting misalignment is not particularly cumbersome when using the method of the present disclosure, as the only detection necessary is a detection of the orientation of the surface of the portion of the surface of the piece of equipment. This can be done using relatively inexpensive imaging systems and imaging systems that can be employed essentially at any time, i.e., during, before, or after use of the pieces of equipment and with or without a person present in the surroundings of the pieces of equipment. For example surface cameras or optical scanners may be used. Thus, misalignment can be detected in a more timely manner than with other, more cumbersome, methods, allowing for faster intervention and adjusting of alignment, thereby also allowing for improving overall alignment over an extended period of time. The method also allows for tracking the alignment of the pieces of equipment over time accurately and with relatively little effort.

In the present disclosure, the medical system may be a system for imaging of objects, like a phantom, and/or subjects, like a patient, and/or for irradiating objects and/or subjects. Subjects and objects are collectively referred to in the present disclosure as "subject".

In the present disclosure, the pieces of equipment may comprise a linear accelerator providing a treatment beam, in particular comprising a gantry and a beam source attached to the gantry, and/or one or more cameras, for example comprising a surface camera, and/or a scanner, and/or an x-ray detector, e.g. a flat panel, and/or an x-ray source for providing x-ray radiation, and/or a platform or support unit, also referred to as patient support or couch, for supporting a subject during operation of the medical system.

The surface of the piece of equipment may be, as an example only, the surface of a housing of the piece of equipment and/or the surface of a support structure of the piece of equipment, e.g., a rack, frame, and/or mount.

In the present disclosure, the predetermined portion of the surface of the piece of equipment may be a point or an area of the surface. The predetermined portion may serve to determine an origin of the normal. In case of a flat surface, the normal will have the same direction irrespective of the origin. However, when the surface is curved, the origin needs to be defined, as the direction of the normal varies depending on where on the surface the origin is located.

In the present disclosure, the viewing direction of the piece of equipment is described by the normal vector, i.e., a vector extending from the predetermined portion of the surface parallel to the normal, specifically from an origin, as mentioned above.

It is noted that the feature "the viewing direction being a normal to a predetermined portion of the surface of the piece of equipment" specifies the meaning of the term "viewing direction". In other words, the viewing direction of the piece of equipment is a normal to a predetermined portion of the surface of the piece of equipment. As such, when a piece of equipment is arranged in space, its viewing direction can be determined by determining said normal.

In the present disclosure, the viewing direction of a piece of equipment is to be seen as the actual viewing direction or as the determined (during the determining) viewing direction, unless specified otherwise. The actual/determined viewing direction may be seen as the viewing direction of the piece of equipment in a given spatial arrangement. The actual/determined viewing direction is to be distinguished from the target viewing direction, which is a theoretical viewing direction. The target viewing direction can also be seen as a target normal to the predetermined portion of the surface of the piece of equipment. The actual/determined viewing direction may coincide with the target viewing direction when the piece of equipment has ideal alignment.

In other words, in view of the above, as an example the present disclosure may be seen as providing a computer-implemented method for use in aligning pieces of equipment of a medical system. The method comprises determining, for a piece of equipment of a medical system, a(n) (actual) viewing direction of the piece of equipment, wherein the (actual) viewing direction is a(n) (actual) normal to a predetermined portion of the surface of the piece of equipment. The method further comprises determining whether the (actual) viewing direction is within a predetermined interval around a target viewing direction of the piece of equipment, and, upon determining that the (actual) viewing direction is outside of the predetermined interval around the target viewing direction of the piece of equipment, initiating an adjusting of the alignment of the piece of equipment.

In other words, in view of the above, as an example the present disclosure may be seen as providing a computer-implemented method for use in aligning pieces of equipment of a medical system. The method comprises determining, for a piece of equipment of a medical system, a viewing direction of the piece of equipment, wherein the (determined) viewing direction is a normal to a predetermined portion of the surface of the piece of equipment. The method further comprises determining whether the determined viewing direction, in other words, the viewing direction determined by the determining, is within a predetermined interval around a target viewing direction of the piece of equipment, and, upon determining that the determined viewing direction is outside of the predetermined interval around the target viewing direction of the piece of equipment, initiating an adjusting of the alignment of the piece of equipment. The target viewing direction may be seen as a target normal to the predetermined portion of the surface of the piece of equipment.

In view of the above, in yet other words, the present disclosure may for example be seen as providing a computer-implemented method for use in aligning pieces of equipment of a medical system. The method comprises determining, for a piece of equipment of a medical system, a(n) (actual) normal to a predetermined portion of the surface of the piece of equipment, in particular, wherein the (actual) normal represents a(n) (actual) viewing direction of the piece of equipment. The method further comprises determining whether the (actual) normal is within a predetermined interval around a target viewing direction of the piece of equipment, and, upon determining that the (actual) normal is outside of the predetermined interval around the target viewing direction of the piece of equipment, initiating an adjusting of the alignment of the piece of equipment. The target viewing direction may be seen as a target normal to the predetermined portion of the surface of the piece of equipment.

In the present disclosure, the target viewing direction indicates the viewing direction in case of a piece of equipment being arranged in a target alignment of the piece of equipment. It may, for example, also be described by a vector. This vector could be seen as a normal vector extending from a plane oriented the way the predetermined portion of the surface of the piece of equipment should be oriented.

The target viewing direction may have a predetermined arrangement relative to an isocenter of a piece of equipment of the medical system, for example an isocenter of a linear accelerator, which may indicate a crossing of the treatment beams for different angular arrangements of the beam source of the linear accelerator, and/or an isocenter of a support unit, which may indicate a point on the rotation axis around which the support unit is pivotable. The point on the rotation axis around which the support unit is pivotable is also referred to, for the sake of brevity, as a rotation point in the present disclosure. This point may be aligned with the isocenter of the linear accelerator such that a pivoting the support unit does not change a relative position of the isocenter of the linear accelerator and the isocenter of the support unit.

In the present disclosure, the predetermined interval around the target viewing direction may describe a tolerance or, in other words, a deviation from the target viewing direction that is considered admissible. The interval may be an angular interval representing an admissible tilt of the viewing direction relative to the target viewing direction. The predetermined interval may, for example, be represented by one or more predetermined thresholds, e.g., a threshold for an angular deviation of the viewing direction from the target viewing direction.

Determining that the viewing direction is outside of the predetermined interval may comprise determining whether a quantitative parameter, e.g., a tilt angle, associated with the viewing direction exceeds a predetermined threshold representative of the predetermined interval, for example a threshold for an angular deviation. The nature and size of the predetermined interval may depend on the specific technical use case and context, e.g., the piece of equipment in question, a required accuracy for a given piece of equipment and/or use case, and/or an overall setup of the medical device, among others. Accordingly, the skilled person would set the predetermined interval to suit the use case and context.

In the present disclosure, initiating an adjusting of the alignment of the piece of equipment may comprise triggering an automatic re-alignment of the piece of equipment, for example by means of a drive system, e.g., a motor. Alternatively or in addition, initiating an adjusting of the alignment of the piece of equipment may comprise triggering outputting an indication to a user that the alignment is outside of the predetermined interval and/or that an adjusting of the piece of equipment is required, in particular, outputting a prompt to the user to adjust the alignment.

Adjusting the alignment may comprise changing the alignment of the piece of equipment so as to bring the viewing direction closer to, particularly within, the predetermined interval around the target viewing direction. Adjusting the alignment may be done by a user manually or by controlling a drive system, e.g. a motor making use of information on the alignment provided on an augmented reality device.

The method of the present disclosure may comprise tracking the alignment of the piece of equipment, the tracking comprising repeatedly determining, for example at predetermined times and/or time intervals or continuously, whether the viewing direction is within the predetermined interval around the target viewing direction of the piece of equipment.

That is, the method may comprise tracking the alignment of the pieces of equipment over time. The method of the present disclosure allows to determine alignment frequently or even continuously, thereby allowing for overall improved accuracy. This can be achieved with relatively little effort. Tracking the alignment may be advantageous in terms of detecting causes for misalignment and/or for determining that a preventative action even prior to an actual occurrence of a misalignment (i.e., outside of the predetermined interval) may be advisable. Furthermore, it may allow for taking action already when misalignments are small, which makes correction easier.

The tracking may comprise monitoring whether and/or how much the alignment changes over time, particularly, whether the actual alignment remains within an acceptable range compared to a target alignment. The tracking may also comprise determining whether the viewing direction continues moving away from the target viewing direction and issue a warning, even when the viewing direction is still within the predetermined interval. For example, the warning may be issued when the viewing direction continues moving away at a rate that exceeds a predetermined rate. The rate may represent the speed of the alignment of the piece of equipment drifting away from the target alignment.

The method, in particular the determining whether the viewing direction is within the predetermined interval, may comprise determining whether a line extending in the viewing direction through a predetermined point on the portion of the surface of the piece of equipment also extends through at least one other predetermined point, wherein the other predetermined point may be a predetermined point on a surface of another piece of equipment and/or a room isocenter and/or an isocenter of another piece of equipment.

Alternatively or in addition, the method, in particular the determining whether the viewing direction is within the predetermined interval, may comprise determining whether a/the line extending in the viewing direction through a/the predetermined point on the portion of the surface of the piece of equipment also extends through at least one predetermined region, wherein the predetermined region may be a predetermined portion of a surface of another piece of equipment and/or a predetermined region in which a/the room isocenter and/or an/the isocenter of another piece of equipment is located.

Thus, for example, the method may comprise determining whether a piece of equipment faces a certain point and/or region, which is often one of the aims of alignment of the pieces of equipment, e.g., pieces of equipment facing an isocenter of the medical system and/or of another one of the pieces of equipment.

The predetermined point may be a point of interest and/or the predetermined region may be a region of interest and determining whether the viewing direction is within the predetermined interval, may comprise determining whether the normal extends through the point of interest and/or the region of interest.

The predetermined interval may, for example, be predetermined such that the viewing direction has a predetermined alignment relative to the point of interest and/or the region of interest. In particular, the allowed misalignment may be determined with respect to the function of the piece of equipment. For example, when the function of a piece of equipment is to take an image of an entire region of interest, the allowed misalignment may be determined such that the piece of equipment provides an image of the entire region of interest.

As an example, the predetermined point or point of interest may be a, particularly reconstructed, isocenter of a linear accelerator, particularly the gantry thereof. A predetermined region or region of interest may be, for example, an area around and comprising the isocenter. As an example, the piece of equipment may be a camera or surface scanner. Thus, by the above methods, the camera or surface scanner can be aimed at the isocenter.

Optionally, an indication, e.g., a visual representation, of the predetermined point and/or the predetermined region and the viewing direction may be provided, e.g., displayed, by an augmented reality device. **In** that case, for example, adjusting of the alignment may be performed by a user manually or by controlling a drive system, e.g. a motor, based on whether or not the indication of the viewing direction, as provided on an augmented reality device extends through the predetermined point and/or region as provided in the augmented reality device. Thus, it allows for a very easy and intuitive way to prompt a user to adjust the alignment, thereby increasing probability that alignment will be adjusted and, accordingly, increasing the overall accuracy.

**In** the present disclosure, the predetermined point and/or the predetermined region may be arranged in a predetermined arrangement relative to at least one marker and determining whether the line extends through the predetermined point and/or the predetermined region may comprise determining the relative arrangement of the line relative to the marker, and deriving the arrangement of the predetermined point and/or the predetermined region relative to the line from the relative arrangement of the line relative to the marker and from the predetermined arrangement of the predetermined point and/or the predetermined region relative to the marker.

Accordingly, determining whether or not the viewing direction is within the predetermined interval may comprise detecting one or more markers indicating the predetermined point and/or predetermined region. A relatively easily detection of whether or not the viewing direction is within the predetermined interval is thus enabled. In other words, the marker can be used to determine whether the piece of equipment faces the predetermined point or region correctly.

It is not necessary that the marker or markers coincide with the predetermined point, e.g. the point of interest, or mark the boundary of the predetermined region, e.g. the region of interest. It is sufficient that the relative position of the marker(s) and the predetermined point or region is known.

The at least one marker may be applied to and/or projected onto an object, for example a piece of equipment, a wall, a ceiling, and/or a floor of the room. For example, one or more lasers may be projected onto the object and the projection may serve as a marker. Alternatively or in addition, a marker, particularly a marker device as known in the art, may be attached to the object or a marker may be printed or drawn onto the object.

The term "marker" is used in the present disclosure to refer collectively to markers and marker devices as defined in the definition section of the present application.

The determining whether or not the viewing direction is within the predetermined interval may, optionally, be a marker-free method. That is, in some examples no markers are used for the determining whether or not the viewing direction is within the predetermined interval, specifically, for detecting the viewing direction, and/or for detecting the portion of the surface. For example, a portion of a surface may be detected based on shape or features of the surface, obtained, for example, using image data of the surface obtained using a surface camera. For example, the surface may have protrusions and/or recesses and/edges that may be used to detect a portion of a surface. Thus, the portion of the surface may be detected even without a marker.

The method of the present disclosure may comprise determining a pivot point of a/the piece of equipment of the medical system. Determining the pivot point may comprise determining a first viewing direction of the piece of equipment when the piece of equipment is in a first arrangement and a second viewing direction of the piece of equipment when the piece of equipment has been pivoted so as to be in a second arrangement. Determining the pivot point may further comprise determining a crossing point of a first line extending in the first viewing direction through a predetermined point on the portion of the surface of the piece of equipment and a second line extending in the second viewing direction through the predetermined point on the portion of the surface of the piece of equipment, and identifying the crossing point to be the pivot point.

For example, the pivot point of a support unit may be determined. This is advantageous as it allows for more precise determination and/or adjustment of the arrangement of an object placed on the support unit relative to other pieces of equipment even when pivoting the support unit.

Optionally, an indication of the pivot point or a pivot axis through the pivot point may be shown in the augmented reality device. The indication may be any visual representation of the pivot point or pivot axis.

The method may comprise a user manually pivoting the piece of equipment from the first arrangement to the second arrangement and/or a user controlling pivoting the piece of equipment by means of a drive system, e.g. a motor, or automatically controlled pivoting of the piece of equipment by means of a drive system, e.g. a motor.

The method of the present disclosure may comprise determining whether an actual path traversed by a/the piece of equipment of the medical system corresponds to a target path to be traversed by the piece of equipment. Determining whether the actual path corresponds to the target path may comprise sending a control signal to a drive system, the control signal instructing the drive system to drive the piece of equipment so as to traverse the target path. Determining whether the actual path corresponds to the target path may further comprise determining the viewing directions of the piece of equipment at multiple arrangements of the piece of equipment along an actual path, which the piece of equipment, driven by the drive system in response to receiving the control signal, traverses.

Determining whether the actual path corresponds to the target path may further comprise determining, for each of the multiple arrangements, whether a line extending along the viewing direction through a predetermined point on the surface of the piece of equipment also extends through a predetermined point and/or region, in particular, whether it intersects with a line extending along the viewing direction through the predetermined point on the surface of the piece of equipment of one or more other arrangements. Determining whether the actual path corresponds to the target path may further comprise, if this is the case, determining that the actual path traversed by the piece of equipment corresponds to the target path to be traversed by the piece of equipment, and otherwise, determining that there is a deviation between the actual path traversed by the piece of equipment and the target path to be traversed by the piece of equipment.

As an example, the target path may be a circle or circular arc. For example, the piece of equipment may be a gantry of a linear accelerator and deviation between the actual path traversed and the target path may be caused by the sag of the gantry.

The deviation between the actual path and the target path may be used for performing corrective actions on the piece of equipment or other pieces of equipment of the medical system. For example, the method may comprise aligning other pieces of equipment so as to account for the deviation.

Alternatively or in addition, the actual path and/or deviation from the target path may be used directly, e.g., for alignment and/or corrective actions, and/or may be used to derive data for performing corrective processing of measurement data obtained by means of the piece of equipment, for example for image reconstruction.

Alternatively or in addition, information that is associated with the actual path may be used for adjusting operation of the medical system.

For example, when there is a deviation between the actual path and the target path of a piece of equipment in the form of a beam source, which may be attached to a gantry, a location and orientation of the beam may deviate. The arrangement and/or operation of the medical system may be adjusted to take this into account, for example, other pieces of equipment of the medical system that are aligned with a target location and orientation of the beam may be re-aligned with the actual location and orientation of the beam. Similarly, when performing imaging with a moving piece of equipment, reconstruction of the images will also depend on the actual path of the piece of equipment.

The above exemplifies that determining the actual path and/or deviations from a target path allows for improved operation, in particular improved accuracy, of the medical system.

An indication of the actual path and optionally an indication of the target path may be show in a/the augmented reality device, particularly alongside an indication of the isocenter. The indication may be any visual representation of the path or the isocenter, respectively. The indication may be overlayed with the field of view of the augmented reality device.

The method may further comprise, when it is determined that there is a deviation between the actual path traversed by the piece of equipment and the target path to be traversed by the piece of equipment, determining more information concerning the deviation, for example by determining the viewing directions for additional arrangements and/or by determining the deviation of the actual line extending along the viewing direction from the target line extending along the viewing direction for each of the arrangements.

This allows for improved accuracy in detecting the actual path and, consequentially, overall improved accuracy of the medical system, as explained above.

For example, when an approximation of the actual path is determined based on a first set of different arrangements of the piece of equipment and it is determined that there is a deviation between the actual path and the target path, data for a second set of arrangements may be obtained, and the determination of the determination of the actual path may be refined using the second set of arrangements. It is to be understood that the second set of arrangements comprises arrangements that are different from those of the first set of arrangements. For example, when the target path is a circle or part of a circle, the first set of different arrangements may be arrangements that differ by a first angle, for example 45°. When it is determined that the actual path is not circular, based on these arrangements, data for a second set of different arrangements that differ by a second angle that is smaller than the first angle, for example 10°, may be obtained. Thus, the determination of the actual path can be refined.

The method of the present disclosure may comprise determining, for a/the piece of equipment of the medical system, an isocenter of the piece of equipment. Determining the isocenter may comprise determining a third viewing direction of the piece of equipment when the piece of equipment is in a third arrangement and a fourth viewing direction of the piece of equipment when the piece of equipment is in a fourth arrangement, wherein each arrangement is one of a plurality of working positions of the piece of equipment. Determining the isocenter may further comprise determining a crossing of a third line that extends in the third viewing direction through a predetermined point on the portion of the surface of the piece of equipment and a fourth line that extends in the fourth viewing direction through the predetermined point on the portion of the surface of the piece of equipment.

For example, a piece of equipment, e.g. a linear accelerator gantry, may be driven through a full rotation and the isocenter may be reconstructed from the crossing of the respective viewing directions of the gantry at different rotation angles, for example such that the arrangements are evenly distributed over the course of the rotation either by angle or by distance. In the case of the gantry, the crossing of the viewing directions may correspond to the isocenter.

The method may also comprise determining a common isocenter of a plurality of pieces of equipment by performing the above method for each of the pieces of equipment. For example, an isocenter may be reconstructed from a crossing of one or more viewing directions of each of the pieces of equipment.

The method of the present disclosure may comprise determining, for each of a plurality of pieces of equipment, the isocenter and/or the pivot point. The method may further comprise aligning the pieces of equipment in such a manner that the isocenter and/or the pivot point of one of the pieces of equipment aligns with the isocenter and/or the pivot point of another of the pieces of equipment.

In particular, the isocenter of a patient support unit, e.g. a couch, which may coincide with the axis of rotation of the patient support unit, may be aligned with the isocenter of the gantry, which may coincide with a treatment beam isocenter.

The above allows for proper alignment of the pieces of equipment. Proper alignment allows for the pieces of equipment to cooperate correctly and precisely, thereby allowing for improved overall accuracy.

In the present disclosure, adjusting the alignment of the piece of equipment may comprise changing the arrangement of the piece of equipment in such a manner that an improvement of the alignment is expected.

The adjusting may be performed fully automatically by means of a drive, e.g., a motor, controlled by a controller or performed in a manner controlled by a user, e.g., by the user controlling operation of a drive, e.g., a motor, or performed manually by a user. Whether an improvement of the alignment is expected can be determined by determining whether changing the arrangement is expected to bring the actual alignment of the piece of equipment closer to a predetermined target alignment.

The method of the present disclosure may comprise, after an arrangement of the piece of equipment has been changed, for example in the course of adjusting the alignment of the piece of equipment, determining whether the alignment has improved. Determining whether the alignment has improved may comprise determining the viewing direction of the piece of equipment and determining whether the viewing direction is within the predetermined interval around the target viewing direction of the piece of equipment and/or verifying the alignment by an alignment method that does not rely on determining the viewing direction.

For example, methods for verifying the alignment that do not rely on determining the viewing direction may be methods that involve imaging, by means of one or more of the pieces of equipment, a phantom of known shape, so as to obtain one or more images of the phantom, and determining, from the images, whether the pieces of equipment are properly aligned. Alternatively, the same procedure may be performed on a subject. The shape of the subject or phantom may be known from a model and/or prior measurements and/or prior and/or concurrent measurements using one or more additional measurement devices.

For example, X-ray imaging of the phantom or the subject may be performed after changing the arrangement and compared to previous X-ray images or MRT/CT images or portal images or prior and/or concurrent images from an optical surface scanner.

Although such a verification may be more cumbersome than the verification by means of determining the viewing direction, accuracy can be improved even further when using different and preferably independent measurement methods. Moreover, due to generally monitoring the alignment using the viewing direction, the more cumbersome methods are not required for tracking alignment but can be used more infrequently, for example for adjusting the alignment to a very high degree of accuracy. For example, the method may comprise verifying the alignment by determining the viewing direction as a default and use other methods only in exceptional situations, which may be predetermined exceptional situations or situations that a user deems exceptional. Thus, undue effort can be avoided.

In the present disclosure, determining the viewing direction of the piece of equipment may comprise analyzing data obtained by a surface camera observing the portion of the surface of the piece of equipment so as to determine the normal vectors of the surface, for example by means of segmentation of a pointcloud detected by the surface camera. A surface camera is an optical camera configured to obtain 3D coordinates of a surface. Surface cameras are often used in medical systems and, accordingly, this may allow for making use of pre-existing system components. Accordingly, no additional hardware and setup is required and compatibility with many different systems can be easily achieved.

Alternatively or in addition, determining the viewing direction of the piece of equipment may comprise analyzing data obtained, for example by a camera, by detecting a marker, arranged on the surface of the piece of equipment in a predetermined arrangement relative to the portion of the surface of the piece of equipment. In particular, the marker may be arranged at the portion of the surface.

Markers are usually inexpensive, widely compatible with different types of equipment, can be easily attached, and image processing for detecting markers is a well-understood technology. Accordingly, using markers is reliable and inexpensive.

The marker may be configured so as to allow for direct calculation of the viewing direction, e.g., normal of the surface to which the marker is attached.

As an example, markers may comprise crosses. Alternatively or in addition, a QR code may be used as a marker. The QR code may comprise information on the piece of equipment or other information relating to the medical system. Thus, it can serve multiple purposes at once. Alternatively or in addition, an Aruco marker may be used for 6D alignment. The Aruco marker is a marker that is specifically configured for determining distance and orientation of the marker and, accordingly, the surface to which it is attached relative to an imaging device that images the marker.

The method of the present disclosure may comprise overlaying an indication of the viewing direction, in particular one or more of the lines of the preceding claims, as part of a virtual image onto the field of view of an augmented reality device. For example, this may be the case when the field of view comprises the piece of equipment. The indication may be any visual representation of the viewing direction, for example a line.

The augmented reality device may, for example, comprise a head mounted device (HMD) that overlays a virtual image with the reality as viewed by the user, e.g., in the form of smart glasses. Alternatively or in addition, the augmented reality device may comprise a device that overlays a virtual image with an image as captured by a camera that may be part of the augmented reality device or a camera external to the augmented reality device.

The field of view may thus be the field of view of a user of the AR device or the field of view of the camera.

As an example an indication of the viewing direction of each of one or more, in particular all of, the flat panels, a camera, a couch, and the gantry may be overlayed onto the field of view. The indication may be any visual representation of said elements.

The indication of the viewing direction or directions, in particular one or more of the lines of the preceding claims, overlayed onto the field of view of an augmented reality device, may be updated continuously or at predetermined intervals. Accordingly, the user can track the arrangement of the pieces of equipment, in particular their alignment, in real time.

The method of the present disclosure may further comprise the step of overlaying an indication of a target viewing direction, in particular one or more target lines, as virtual image onto the field of view of the augmented reality device, in particular at the same time as the indication of the viewing direction. The indication may be any visual representation of the target viewing direction or viewing direction, e.g., a line.

The advantage of overlaying the indications of viewing directions onto the field of view of an augmented reality device is advantageous because determining whether or not to actually intervene and, if so, how to intervene in case of misalignment may be computationally expensive and complex, such that it may be preferable to have a user to decide or at least check a decision made by a computer. The technical information required for this decision can be made available to the user particularly efficiently, e.g., in terms of computing resources, by using the augmented reality device as specified above. That is, the field of view of the augmented reality device - without overlaying it with a virtual image - already by default comprises most of the information required by the user, and only little additional information needs to be added (e.g., overlayed onto the field of view) to provide the full set of required technical information. Thus, the method allows for decisions regarding potential correction of alignment being made reliably and efficiently.

The method of the present disclosure may comprise providing an indication to a user of an/the augmented reality device when the viewing direction approaches and/or diverges from a/the target viewing direction, in particular, prompting the user to initiate verifying and/or adjusting the alignment, for example, when the viewing direction diverges from the target viewing direction by more than a predetermined rate and/or amount.

The prompt may be a visual prompt overlayed onto the field of view and/or an audio prompt and/or a haptic prompt.

Similarly to the above considerations, this also allows for decisions regarding potential correction of alignment being made reliably and efficiently. For example, a computing device may efficiently and reliably determine whether the viewing direction approaches or diverges from the target viewing direction, but it may be less efficient and reliable when it comes to deciding whether and how to verify or adjust the alignment. Thus, prompting the user to decide allows for high efficiency and reliability.

The method of the present disclosure may comprise accessing alignment data from an alignment or an adjustment of the alignment of the piece of equipment, the alignment or adjustment of alignment making use of a method not relying on determining the viewing direction. The method may further comprise subsequently, determining the viewing direction of the piece of equipment, and determining whether there is a discrepancy between the alignment performed prior to determining the viewing direction and an alignment derived from the viewing direction. The method may comprise, if it is determined that there is a discrepancy, performing a calibration of the method used for determining the viewing direction of the piece of equipment so as to reduce the discrepancy, in particular such that there is no discrepancy. The method may further comprise, only after determining that there is no discrepancy, performing the step of, upon determining that the viewing direction is outside of the predetermined interval around the target viewing direction of the piece of equipment, initiating the adjusting of the alignment of the piece of equipment.

In other words, prior to using the viewing direction for determining whether adjusting the alignment should be initiated, the accuracy of the method for determining alignment by means of the viewing direction may be verified by comparing its results with the results of a method that does not employ detection of the viewing direction. This allows for improving overall accuracy of the method based on the viewing direction in reliably detecting misalignments and reducing the amount of false positive detections of misalignments.

Any steps mentioned in the present disclosure as part of the method that are not carried out by a computing system may be carried out manually by a user and/or by one of the pieces of equipment, optionally controlled by a computing system or by a user input. It is noted that the terms computer and computing system are used interchangeably in the present disclosure.

The present disclosure also provides a medical system comprising at least one piece of equipment, and a computing system configured to carry out and/or control any of the method steps of the present disclosure, particularly any of the above-described methods, unless they are specified as being carried out or controlled only by a user or only by another component or cannot be carried out or controlled by a computing system.

The medical system of the present disclosure may comprise one or more imaging devices, in particular a surface camera, configured to obtain image data of the piece of equipment, in particular of the portion of the surface of the piece of equipment and/or of a/the marker and provide the image data to the computing system, wherein the computing system is configured to determine the viewing direction of the piece of equipment based on the image data.

Alternatively or in addition, the medical system of the present disclosure may comprise a drive system, for example a motor, configured to drive the piece of equipment in response to receiving a/the control signal to drive the piece of equipment so as to traverse a/the target path.

Alternatively or in addition, the medical system of the present disclosure may comprise an/the augmented reality device, in particular, wherein at least one of the one or more imaging devices mentioned above is comprised in or connected to the augmented reality device.

The at least one piece of equipment of the present disclosure may comprise at least one of: a linear accelerator for creating a beam, one or more panels for detecting X-ray radiation, an X-ray source, one or more cameras, a support unit for supporting a subject, in particular, a subject to be placed into a/the beam of a/the linear accelerator.

The computing system of the present disclosure may be configured to issue control signals to one or more pieces of equipment of the medical system and/or to the drive system and/or the augmented reality device, in particular control signals to prompt the one or more imaging devices to obtain data and/or to prompt the augmented reality device to perform the overlaying of the indication of the viewing direction and/or to prompt the drive system to drive a piece of equipment. As explained elsewhere, however, a processing system of the augmented reality device may also be may be part of the computing system.

The medical system of the present disclosure may comprise a radiation treatment apparatus comprising a treatment beam source and a patient support unit, wherein the computing system is operably coupled to the radiation treatment apparatus for issuing a control signal to the radiation treatment apparatus for controlling the operation of the treatment beam source and/or the position of the patient support unit.

For example, the treatment beam source may be one of the pieces of equipment of the medical system. The treatment beam source may be attached to a rotatable gantry. The rotatable gantry may be one of the pieces of equipment of the medical system. Since the beam source may be attached to the gantry, they may also together be seen as a piece of equipment of the medical system. The patient support unit may be one of the pieces of equipment, and in particular, may be pivotable around an isocenter. The patient support unit may be a couch.

The present disclosure also provides a computer program which, when the program is executed by a computer or when loaded onto a computer, causes the computer to carry out and/or control any of the method steps of the present disclosure, in particular, any of the above-described method steps, unless specified as being performed by a user.

The present disclosure also provides a program storage medium on which the computer program is stored.

The present disclosure also provides a computer comprising at least one processor and a memory and/or the program storage medium, wherein the program is running on the computer or loaded into the memory of the computer. The present disclosure also provides a signal wave or a digital signal wave, carrying information which represents the program. The present disclosure also provides a data stream which is representative of the program.

The present invention also relates to the use of the medical system for positioning a subject, for example by pivoting or otherwise moving the support unit of the medical system according to the present disclosure and/or imaging of a subject for example by means of X-ray imaging pieces of equipment of the medical system according to the present disclosure, and/or irradiating a subject, for example with a beam from the beam source of the medical system according to the present disclosure.

For example, the invention does not involve or in particular comprise or encompass an invasive step which would represent a substantial physical interference with the body requiring professional medical expertise to be carried out and entailing a substantial health risk even when carried out with the required professional care and expertise. For example, the invention does not comprise a step of positioning a medical implant in order to fasten it to an anatomical structure or a step of fastening the medical implant to the anatomical structure or a step of preparing the anatomical structure for having the medical implant fastened to it. More particularly, the invention does not involve or in particular comprise or encompass any surgical or therapeutic activity. The invention is instead directed as applicable to reliably and precisely setting up of pieces of equipment and allowing for ensuring continued proper setup thereof. For this reason alone, no surgical or therapeutic activity and in particular no surgical or therapeutic step is necessitated or implied by carrying out the invention.

The features and advantages outlined above in the context of the method similarly apply to the medical system, computer program, program storage medium, computer, signal wave, data stream and use of the medical system described in the present disclosure.

### DEFINITIONS

In this section, definitions for specific terminology used in this disclosure are offered which also form part of the present disclosure.

### Computer implemented method

Some of the methods of the present disclosure are for example a computer implemented method. For example, all the steps or merely some of the steps (i.e. less than the total number of steps) of the method in accordance with the invention can be executed by a computer (for example, at least one computer). An embodiment of the computer implemented method is a use of the computer for performing a data processing method. An embodiment of the computer implemented method is a method concerning the operation of the computer such that the computer is operated to perform one, more or all steps of the method.

The computer for example comprises at least one processor and for example at least one memory in order to (technically) process the data, for example electronically and/or optically. The processor being for example made of a substance or composition which is a semiconductor, for example at least partly n- and/or p-doped semiconductor, for example at least one of II-, III-, IV-, V-, VI-semiconductor material, for example (doped) silicon and/or gallium arsenide. The calculating or determining steps described are for example performed by a computer. Determining steps or calculating steps are for example steps of determining data within the framework of the technical method, for example within the framework of a program. A computer is for example any kind of data processing device, for example electronic data processing device. A computer can be a device which is generally thought of as such, for example desktop PCs, notebooks, netbooks, etc., but can also be any programmable apparatus, such as for example a mobile phone or an embedded processor. A computer can for example comprise a system (network) of "sub-computers", wherein each sub-computer represents a computer in its own right. The term "computer" includes a cloud computer, for example a cloud server. The term "cloud computer" includes a cloud computer system which for example comprises a system of at least one cloud computer and for example a plurality of operatively interconnected cloud computers such as a server farm. Such a cloud computer is preferably connected to a wide area network such as the world wide web (WWW) and located in a so-called cloud of computers which are all connected to the world wide web. Such an infrastructure is used for "cloud computing", which describes computation, software, data access and storage services which do not require the end user to know the physical location and/or configuration of the computer delivering a specific service. For example, the term "cloud" is used in this respect as a metaphor for the Internet (world wide web). For example, the cloud provides computing infrastructure as a service (IaaS). The cloud computer can function as a virtual host for an operating system and/or data processing application which is used to execute the method of the invention. The cloud computer is for example an elastic compute cloud (EC2) as provided by Amazon Web Services^{™}. A computer for example comprises interfaces in order to receive or output data and/or perform an analogue-to-digital conversion. The data are for example data which represent physical properties and/or which are generated from technical signals. The technical signals are for example generated by means of (technical) detection devices (such as for example devices for detecting marker devices) and/or (technical) analytical devices (such as for example devices for performing (medical) imaging methods), wherein the technical signals are for example electrical or optical signals. The technical signals for example represent the data received or outputted by the computer. The computer is preferably operatively coupled to a display device which allows information outputted by the computer to be displayed, for example to a user. One example of a display device is a virtual reality device or an augmented reality device (also referred to as virtual reality glasses or augmented reality glasses) which can be used as "goggles" for navigating. A specific example of such augmented reality glasses is Google Glass (a trademark of Google, Inc.). An augmented reality device or a virtual reality device can be used both to input information into the computer by user interaction and to display information outputted by the computer. Another example of a display device would be a standard computer monitor comprising for example a liquid crystal display operatively coupled to the computer for receiving display control data from the computer for generating signals used to display image information content on the display device. A specific embodiment of such a computer monitor is a digital lightbox. An example of such a digital lightbox is Buzz^{®}, a product of Brainlab AG. The monitor may also be the monitor of a portable, for example handheld, device such as a smart phone or personal digital assistant or digital media player.

The invention also relates to a program which, when running on a computer, causes the computer to perform one or more or all of the method steps described herein and/or to a program storage medium on which the program is stored (in particular in a non-transitory form) and/or to a computer comprising said program storage medium and/or to a (physical, for example electrical, for example technically generated) signal wave, for example a digital signal wave, carrying information which represents the program, for example the aforementioned program, which for example comprises code means which are adapted to perform any or all of the method steps described herein.

Within the framework of the invention, computer program elements can be embodied by hardware and/or software (this includes firmware, resident software, micro-code, etc.). Within the framework of the invention, computer program elements can take the form of a computer program product which can be embodied by a computer-usable, for example computer-readable data storage medium comprising computer-usable, for example computer-readable program instructions, "code" or a "computer program" embodied in said data storage medium for use on or in connection with the instruction-executing system. Such a system can be a computer; a computer can be a data processing device comprising means for executing the computer program elements and/or the program in accordance with the invention, for example a data processing device comprising a digital processor (central processing unit or CPU) which executes the computer program elements, and optionally a volatile memory (for example a random access memory or RAM) for storing data used for and/or produced by executing the computer program elements. Within the framework of the present invention, a computer-usable, for example computer-readable data storage medium can be any data storage medium which can include, store, communicate, propagate or transport the program for use on or in connection with the instruction-executing system, apparatus or device. The computer-usable, for example computer-readable data storage medium can for example be, but is not limited to, an electronic, magnetic, optical, electromagnetic, infrared or semiconductor system, apparatus or device or a medium of propagation such as for example the Internet. The computer-usable or computer-readable data storage medium could even for example be paper or another suitable medium onto which the program is printed, since the program could be electronically captured, for example by optically scanning the paper or other suitable medium, and then compiled, interpreted or otherwise processed in a suitable manner. The data storage medium is preferably a non-volatile data storage medium. The computer program product and any software and/or hardware described here form the various means for performing the functions of the invention in the example embodiments. The computer and/or data processing device can for example include a guidance information device which includes means for outputting guidance information. The guidance information can be outputted, for example to a user, visually by a visual indicating means (for example, a monitor and/or a lamp) and/or acoustically by an acoustic indicating means (for example, a loudspeaker and/or a digital speech output device) and/or tactilely by a tactile indicating means (for example, a vibrating element or a vibration element incorporated into an instrument). For the purpose of this document, a computer is a technical computer which for example comprises technical, for example tangible components, for example mechanical and/or electronic components. Any device mentioned as such in this document is a technical and for example tangible device.

### Marker

It is the function of a marker to be detected by a marker detection device (for example, a camera or an ultrasound receiver or analytical devices such as CT or MRI devices) in such a way that its spatial position (i.e. its spatial location and/or alignment) can be ascertained. The detection device is for example part of a navigation system. The markers can be active markers. An active marker can for example emit electromagnetic radiation and/or waves which can be in the infrared, visible and/or ultraviolet spectral range. A marker can also however be passive, i.e. can for example reflect electromagnetic radiation in the infrared, visible and/or ultraviolet spectral range or can block x-ray radiation. To this end, the marker can be provided with a surface which has corresponding reflective properties or can be made of metal in order to block the x-ray radiation. It is also possible for a marker to reflect and/or emit electromagnetic radiation and/or waves in the radio frequency range or at ultrasound wavelengths. A marker may have a spherical and/or spheroid shape and can therefore be referred to as a marker sphere; markers can however also exhibit a cornered, for example cubic, shape.

### Marker device

A marker device can for example be a reference star or a pointer or a single marker or a plurality of (individual) markers which are then preferably in a predetermined spatial relationship. A marker device comprises one, two, three or more markers, wherein two or more such markers are in a predetermined spatial relationship. This predetermined spatial relationship is for example known to a navigation system and is for example stored in a computer of the navigation system.

In another embodiment, a marker device comprises an optical pattern, for example on a two-dimensional surface. The optical pattern might comprise a plurality of geometric shapes like circles, rectangles and/or triangles. The optical pattern can be identified in an image captured by a camera, and the position of the marker device relative to the camera can be determined from the size of the pattern in the image, the orientation of the pattern in the image and the distortion of the pattern in the image. This allows determining the relative position in up to three rotational dimensions and up to three translational dimensions from a single two-dimensional image.

The position of a marker device can be ascertained, for example by a medical navigation system. If the marker device is attached to an object, such as a bone or a medical instrument, the position of the object can be determined from the position of the marker device and the relative position between the marker device and the object. Determining this relative position is also referred to as registering the marker device and the object. The marker device or the object can be tracked, which means that the position of the marker device or the object is ascertained twice or more over time.

### Treatment beam

The present disclosure may also be applicable for medical systems that are configured to generating and/or controlling a treatment beam. The treatment beam treats body parts which are to be treated and which are referred to in the following as "treatment body parts". These body parts are for example parts of a patient's body, i.e. anatomical body parts.

The present invention relates to the field of medicine and for example to the use of beams, such as radiation beams, to treat parts of a patient's body, which are therefore also referred to as treatment beams. A treatment beam treats body parts which are to be treated and which are referred to in the following as "treatment body parts". These body parts are for example parts of a patient's body, i.e. anatomical body parts. Ionizing radiation is for example used for the purpose of treatment. For example, the treatment beam comprises or consists of ionizing radiation. The ionizing radiation comprises or consists of particles (for example, sub-atomic particles or ions) or electromagnetic waves which are energetic enough to detach electrons from atoms or molecules and so ionize them. Examples of such ionizing radiation include x-rays, high-energy particles (high-energy particle beams) and/or ionizing radiation emitted from a radioactive element. The treatment radiation, for example the treatment beam, is for example used in radiation therapy or radiotherapy, such as in the field of oncology. For treating cancer in particular, parts of the body comprising a pathological structure or tissue such as a tumor are treated using ionizing radiation. The tumor is then an example of a treatment body part.

The treatment beam is preferably controlled such that it passes through the treatment body part. However, the treatment beam can have a negative effect on body parts outside the treatment body part. These body parts are referred to here as "outside body parts". Generally, a treatment beam has to pass through outside body parts in order to reach and so pass through the treatment body part.

Reference is also made in this respect to the following web pages: http://www.elekta.com/healthcare_us_elekta_vmat.php and http://www.varian.com/us/oncology/treatments/treatment_techniques/rapidarc.

### Imaging methods

In the field of medicine, imaging methods (also called imaging modalities and/or medical imaging modalities) are used to generate image data (for example, two-dimensional or three-dimensional image data) of anatomical structures (such as soft tissues, bones, organs, etc.) of the human body. The term "medical imaging methods" is understood to mean (advantageously apparatus-based) imaging methods (for example so-called medical imaging modalities and/or radiological imaging methods) such as for instance computed tomography (CT) and cone beam computed tomography (CBCT, such as volumetric CBCT), x-ray tomography, magnetic resonance tomography (MRT or MRI), conventional x-ray, sonography and/or ultrasound examinations, and positron emission tomography. For example, the medical imaging methods are performed by the analytical devices. Examples for medical imaging modalities applied by medical imaging methods are: X-ray radiography, magnetic resonance imaging, medical ultrasonography or ultrasound, endoscopy, elastography, tactile imaging, thermography, medical photography and nuclear medicine functional imaging techniques as positron emission tomography (PET) and Single-photon emission computed tomography (SPECT), as mentioned by Wikipedia.

The image data thus generated is also termed "medical imaging data". Analytical devices for example are used to generate the image data in apparatus-based imaging methods. The imaging methods are for example used for medical diagnostics, to analyze the anatomical body in order to generate images which are described by the image data. The imaging methods are also for example used to detect pathological changes in the human body. However, some of the changes in the anatomical structure, such as the pathological changes in the structures (tissue), may not be detectable and for example may not be visible in the images generated by the imaging methods. A tumor represents an example of a change in an anatomical structure. If the tumor grows, it may then be said to represent an expanded anatomical structure. This expanded anatomical structure may not be detectable; for example, only a part of the expanded anatomical structure may be detectable. Primary/high-grade brain tumors are for example usually visible on MRI scans when contrast agents are used to infiltrate the tumor. MRI scans represent an example of an imaging method. In the case of MRI scans of such brain tumors, the signal enhancement in the MRI images (due to the contrast agents infiltrating the tumor) is considered to represent the solid tumor mass. Thus, the tumor is detectable and for example discernible in the image generated by the imaging method. In addition to these tumors, referred to as "enhancing" tumors, it is thought that approximately 10% of brain tumors are not discernible on a scan and are for example not visible to a user looking at the images generated by the imaging method.

### Fixed (relative) position

A fixed position, which is also referred to as fixed relative position, in this document means that two objects which are in a fixed position have a relative position which does not change unless this change is explicitly and intentionally initiated. A fixed position is in particular given if a force or torque above a predetermined threshold has to be applied in order to change the position. This threshold might be 10 N or 10 Nm. In particular, the position of a sensor device remains fixed relative to a target while the target is registered or two targets are moved relative to each other. A fixed position can for example be achieved by rigidly attaching one object to another. The spatial location, which is a part of the position, can in particular be described just by a distance (between two objects) or just by the direction of a vector (which links two objects). The alignment, which is another part of the position, can in particular be described by just the relative angle of orientation (between the two objects).

### BRIEF DESCRIPTION OF THE DRAWINGS

In the following, the invention is described with reference to the appended figures which give background explanations and represent specific embodiments of the invention. The scope of the invention is however not limited to the specific features disclosed in the context of the figures, wherein
- Fig. 1: illustrates the steps of a method according to the present disclosure;
- Fig. 2: illustrates steps of determining a linear accelerator's isocenter;
- Fig. 3: is a schematic, not to scale illustration of a medical system according to the present disclosure; and
- Fig. 4: shows a schematic and not to scale view of pieces of equipment of a medical system and an augmented reality device according to the present disclosure.

### DESCRIPTION OF EMBODIMENTS

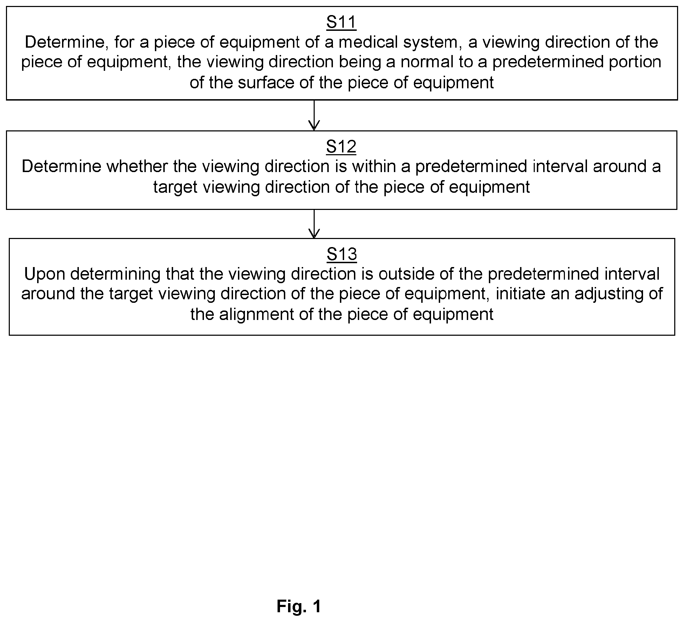

Fig. 1 illustrates the basic steps of a method for use in aligning pieces of equipment of a medical system according to the present disclosure.

Step S11 encompasses determining, for a piece of equipment of a medical system, a viewing direction of the piece of equipment, the viewing direction being a normal to a predetermined portion of the surface of the piece of equipment. For example, a normal on the surface of a gantry of a linear accelerator and/or on the surface of a camera and/or on the surface of a detector and/or on the surface of a patient support. This may be done by means of a surface scan and detecting surface shape and/or features or making use of markers.

Step S12 encompasses determining whether the viewing direction is within a predetermined interval around a target viewing direction of the piece of equipment.

Step S13 encompasses, upon determining that the viewing direction is outside of the predetermined interval around the target viewing direction of the piece of equipment, initiating an adjusting of the alignment of the piece of equipment. An alignment may be performed manually by an user or automatically or semi-automatically. It may comprise rearranging the gantry or camera or detector or patient support, respectively.

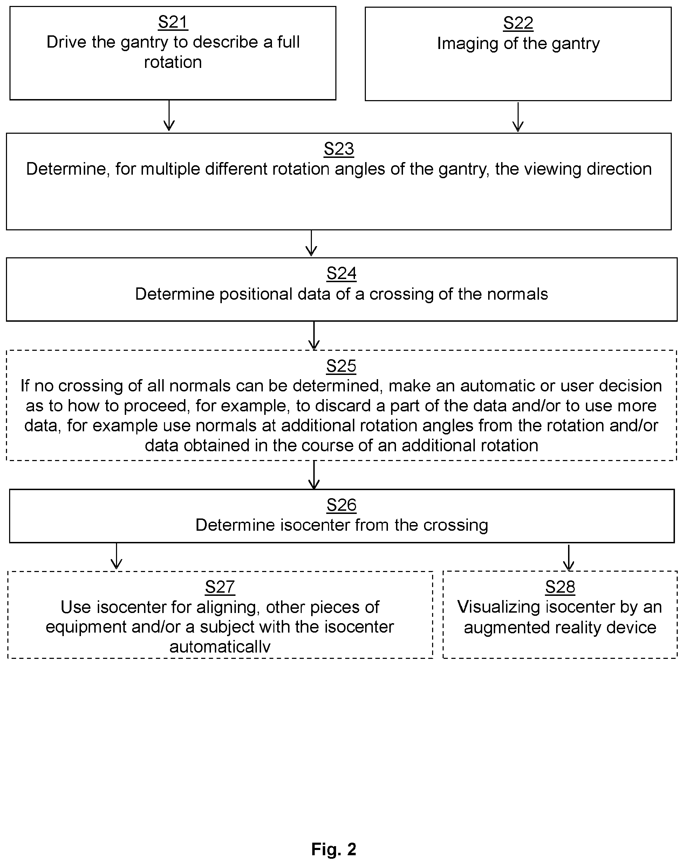

Fig. 2 illustrates the basic steps of determining and optionally using a linear accelerator's isocenter using a viewing direction, e.g., normal on a portion of the surface of the linear accelerator.

The linear accelerator of this example has a gantry to which a beam source is attached. Determining the isocenter comprises driving the gantry to describe a full rotation S21 and, at the same time, imaging S22 the gantry. More specifically, the imaging comprises imaging of a predetermined portion of the surface of the gantry. In step S23, for multiple different rotation angles of the gantry, the viewing direction is determined from the images obtained by the imaging. Specifically, for each rotation angle, the normal to the predetermined portion of the surface is determined from the image data obtained by imaging the gantry.

In step S24, positional data of a crossing of the normals is then determined. Optionally, in step S25, if no crossing of all normals can be determined, an automatic or user decision may be made as to how to proceed, for example, to discard a part of the data and/or to use more data, for example use normals at additional rotation angles from the rotation and/or data obtained in the course of an additional rotation. Depending on the required accuracy, averaging might also be performed to obtain an estimate of the crossing. In step S26, the isocenter is determined from the crossing. The crossing may, for example, correspond to the isocenter.

The normals and/or the isocenter may be visualized by an augmented reality device, e.g., by overlaying an indication of the normals and/or isocenter on the field of view.

A suitable portion of the surface of the gantry may be a surface facing towards the isocenter, i.e., an inward-facing portion of the surface.

The isocenter that results from the above method may, for example, be used for aligning, in step S27, other pieces of equipment and/or a subject with the isocenter automatically, semi-automatically, or manually by a user, for example as described above. Optionally, the isocenter may also be used to be visualized, in step S28, by an augmented reality device, e.g., a head mounted device, for example as described above. The isocenter may be visualized alongside a patient support unit's isocenter and/or a target isocenter for alignment.

Alternatively or in addition, determining a linear accelerator's isocenter, the method of the present disclosure may comprise determining a patient support unit's (also referred to as patient support) isocenter using normals. The patient support's isocenter may, for example, be a point on a rotation axis around which the patient support rotates, which may be a vertical rotation axis. In operation of the medical system, the patient support's isocenter may optionally be aligned with the linear accelerator's isocenter, which may be determined as described above, for example. The rotation axis in many cases is an inner rotation axis. Accordingly, compared to the example of the linear accelerator's isocenter, there is a difference in that the predetermined portion of the surface used for determining the normal will not be facing the isocenter of the patient support. Instead, an outward facing surface is used for determining the normal. However, a crossing of an extension of the normals into the patient support can nonetheless be determined to obtain a crossing and the isocenter.

Thus, essentially the method steps may be the same as with the linear accelerator except that a surface of the patient support is imaged at different rotation angles. Similarly to the linear accelerator isocenter, the isocenter of the patient support may also be used for alignment, for example with the linear accelerator isocenter, and/or may be visualized in an augmented reality device, for example alongside the linear accelerator's isocenter and/or a target isocenter for alignment.

In each of the cases, care must be taken that the predetermined portion of the surface can be properly imaged for each of the rotation angles used for determining the normals. If one detector does not suffice for imaging at each of the rotation image data, image data from a plurality of detectors arranged in fixed positions may be used. Although image data from a moving detector might also be used, this would make the overall determination more difficult, as the calculations would also have to account for the detector movement relative to the portion of the surface.

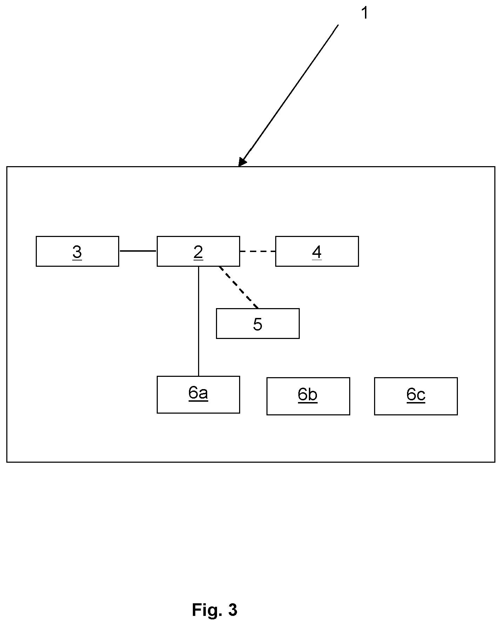

Fig. 3 is a schematic illustration of a medical system 1 according to the present disclosure. The system is in its entirety identified by reference sign 1 and comprises a computing system 2, an electronic data storage device (such as a hard disc) 3, which is here shown as separate to the computing system but may also be part of the computing system and a drive system 4. The system may also comprise an augmented reality device 5. Some or all of the computing system's functions may be carried out by the augmented reality device. E.g., the processing system of the augmented reality device may be part of the computing system. Moreover, several pieces of equipment 6a to 6c are shown merely schematically, the number of pieces of equipment being exemplary only and non-limiting. The components of the medical system 1 may have the functionalities and properties explained above.

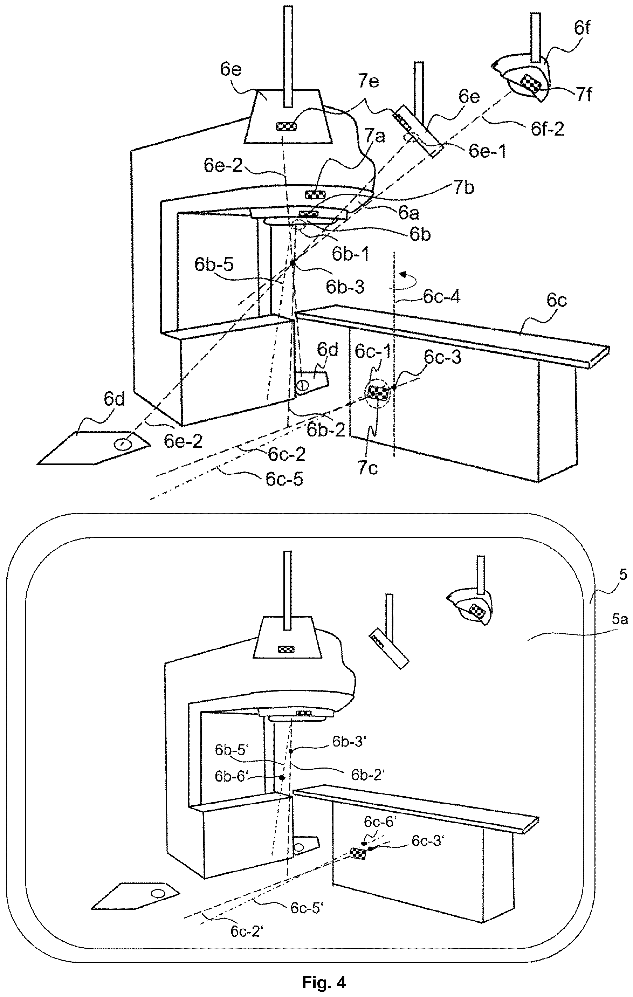

Fig. 4 shows a schematic and not to scale view of some examples and arrangements of pieces of equipment 6a to 6f of a medical system 1 according to the present disclosure.

In particular, the medical system of Fig. 4 comprises a linear accelerator having a gantry 6a and, attached in a fixed relative position to the gantry, a beam source 6b. The medical system further comprises a patient support unit 6c. The medical system further comprises x-ray sources 6d and x-ray detectors 6e, e.g., in the form of flat panels. The medical system may further comprise at least one surface camera 6f.

In the Figure, some examples of the predetermined portions of the surface of some of the pieces of equipment are indicated by reference signs 6b-1, 6c-1, and 6e-1, and the respective normals through the predetermined portions of the surface by reference signs 6b-2, 6c-2, 6e-2, and 6f-2.

Moreover, a first isocenter 6b-3 of the linear accelerator and a second isocenter 6c-3 of the patient support are illustrated in the Figure. Furthermore, a rotation axis 6c-4 of the patient support are illustrated. The isocenters are located on the respective rotation axis in this example.

Markers 7a, 7b, 7c, 7e, and 7f are schematically shown on the pieces of equipment. However, markers need not be used, for example, in case the portion of a surface need not be detected, or a portion of a surface can be detected even without a marker, e.g., based on shape or features of the surface, obtained, for example, using image data of the surface obtained using a surface camera.

Furthermore, merely as an example, the field of view 5a of an augmented reality device 5 overlayed with an indication 6b-2' and 6c-2' of the actual viewing directions of the beam source and the patient support, indications 6b-5' and 6c-5' of the target viewing directions 6b-5 and 6c-5, and indications 6b-3' and 6c-3' of the actual isocenters and 6b-6' and 6c-6' of target isocenters. It is noted that in Fig. 4 the augmented reality device is illustrated at an exaggerated size to properly show the field of view.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims.

## Claims

1. A computer-implemented method for use in aligning pieces of equipment (6a, 6b, 6c, 6d, 6e, 6f) of a medical system (1), the method comprising:
determining (S11), for a piece of equipment (6a, 6b, 6c, 6d, 6e, 6f) of a medical system (1), a viewing direction of the piece of equipment (6a, 6b, 6c, 6d, 6e, 6f), the viewing direction being described by a normal (6b-2, 6c-2, 6e-2, 6f-2) to a predetermined portion of the surface (6b-1, 6c-1, 6e-1) of the piece of equipment (6a, 6b, 6c, 6d, 6e, 6f),
determining (S12) whether the viewing direction is within a predetermined interval around a target viewing direction (6b-5, 6c-5) of the piece of equipment (6a, 6b, 6c, 6d, 6e, 6f), and
upon determining that the viewing direction is outside of the predetermined interval around the target viewing direction (6b-5, 6c-5) of the piece of equipment (6a, 6b, 6c, 6d, 6e, 6f), initiating (S13) an adjusting of the alignment of the piece of equipment (6a, 6b, 6c, 6d, 6e, 6f).

2. The method of claim 1, further comprising tracking the alignment of the piece of equipment (6a, 6b, 6c, 6d, 6e, 6f), the tracking comprising repeatedly determining, for example at predetermined times and/or time intervals or continuously, whether the viewing direction is within the predetermined interval around the target viewing direction (6b-5, 6c-5) of the piece of equipment (6a, 6b, 6c, 6d, 6e, 6f).

3. The method of claim 1 or 2, wherein the method, in particular the determining whether the viewing direction is within the predetermined interval, comprises:
determining whether a line extending in the viewing direction through a predetermined point on the portion of the surface (6b-1, 6c-1, 6e-1) of the piece of equipment (6a, 6b, 6c, 6d, 6e, 6f) also extends through at least one other predetermined point, wherein the other predetermined point may be a predetermined point on a surface of another piece of equipment (6a, 6b, 6c, 6d, 6e, 6f) and/or a room isocenter (6b-3, 6c-3) and/or an isocenter (6b-3, 6c-3) of another piece of equipment (6a, 6b, 6c, 6d, 6e, 6f), and/or
determining whether a/the line extending in the viewing direction through a/the predetermined point on the portion of the surface (6b-1, 6c-1, 6e-1) of the piece of equipment (6a, 6b, 6c, 6d, 6e, 6f) also extends through at least one predetermined region, wherein the predetermined region may be a predetermined portion of a surface of another piece of equipment (6a, 6b, 6c, 6d, 6e, 6f) and/or a predetermined region in which a/the room isocenter (6b-3, 6c-3) and/or an/the isocenter (6b-3, 6c-3) of another piece of equipment (6a, 6b, 6c, 6d, 6e, 6f) is located.

4. The method of claim 3, wherein the predetermined point and/or the predetermined region is arranged in a predetermined arrangement relative to at least one marker (7a, 7b, 7c, 7e, 7f) and determining whether the line extends through the predetermined point and/or the predetermined region comprises:
determining the relative arrangement of the line relative to the marker (7a, 7b, 7c, 7e, 7f), and
deriving the arrangement of the predetermined point and/or the predetermined region relative to the line from the relative arrangement of the line relative to the marker (7a, 7b, 7c, 7e, 7f) and from the predetermined arrangement of the predetermined point and/or the predetermined region relative to the marker (7a, 7b, 7c, 7e, 7f).

5. The method of any of the preceding claims, further comprising determining a pivot point of a/the piece of equipment (6a, 6b, 6c, 6d, 6e, 6f) of the medical system (1),
wherein determining the pivot point comprises:
determining a first viewing direction of the piece of equipment (6a, 6b, 6c, 6d, 6e, 6f) when the piece of equipment (6a, 6b, 6c, 6d, 6e, 6f) is in a first arrangement and a second viewing direction of the piece of equipment (6a, 6b, 6c, 6d, 6e, 6f) when the piece of equipment (6a, 6b, 6c, 6d, 6e, 6f) has been pivoted so as to be in a second arrangement,
determining a crossing point of a first line extending in the first viewing direction through a predetermined point on the portion of the surface (6b-1, 6c-1, 6e-1) of the piece of equipment (6a, 6b, 6c, 6d, 6e, 6f) and a second line extending in the second viewing direction through the predetermined point on the portion of the surface (6b-1, 6c-1, 6e-1) of the piece of equipment (6a, 6b, 6c, 6d, 6e, 6f), and
identifying the crossing point to be the pivot point.

6. The method of any of the preceding claims, further comprising determining whether an actual path traversed by a/the piece of equipment (6a, 6b, 6c, 6d, 6e, 6f) of the medical system (1) corresponds to a target path to be traversed by the piece of equipment,
wherein determining whether the actual path corresponds to the target path comprises:
sending a control signal to a drive system (4), the control signal instructing the drive system (4) to drive the piece of equipment (6a, 6b, 6c, 6d, 6e, 6f) so as to traverse the target path,
determining the viewing directions of the piece of equipment (6a, 6b, 6c, 6d, 6e, 6f) at multiple arrangements of the piece of equipment (6a, 6b, 6c, 6d, 6e, 6f) along an actual path, which the piece of equipment (6a, 6b, 6c, 6d, 6e, 6f), driven by the drive system (4) in response to receiving the control signal, traverses,
determining, for each of the multiple arrangements, whether a line extending along the viewing direction through a predetermined point on the surface of the piece of equipment (6a, 6b, 6c, 6d, 6e, 6f) also extends through a predetermined point and/or region, in particular, whether it intersects with a line extending along the viewing direction through the predetermined point on the surface of the piece of equipment (6a, 6b, 6c, 6d, 6e, 6f) of one or more other arrangements,
if this is the case, determining that the actual path traversed by the piece of equipment (6a, 6b, 6c, 6d, 6e, 6f) corresponds to the target path to be traversed by the piece of equipment (6a, 6b, 6c, 6d, 6e, 6f), and
otherwise, determining that there is a deviation between the actual path traversed by the piece of equipment (6a, 6b, 6c, 6d, 6e, 6f) and the target path to be traversed by the piece of equipment (6a, 6b, 6c, 6d, 6e, 6f).

7. The method of claim 6, further comprising, when it is determined that there is a deviation between the actual path traversed by the piece of equipment (6a, 6b, 6c, 6d, 6e, 6f) and the target path to be traversed by the piece of equipment (6a, 6b, 6c, 6d, 6e, 6f), determining more information concerning the deviation, for example by determining the viewing directions for additional arrangements and/or by determining the deviation of the actual line extending along the viewing direction from the target line extending along the viewing direction for each of the arrangements.

8. The method of any of the preceding claims, further comprising determining, for a/the piece of equipment (6a, 6b, 6c, 6d, 6e, 6f) of the medical system (1), an isocenter (6b-3, 6c-3) of the piece of equipment (6a, 6b, 6c, 6d, 6e, 6f),
wherein determining the isocenter (6b-3, 6c-3) comprises:
determining a third viewing direction of the piece of equipment (6a, 6b, 6c, 6d, 6e, 6f) when the piece of equipment (6a, 6b, 6c, 6d, 6e, 6f) is in a third arrangement and a fourth viewing direction of the piece of equipment (6a, 6b, 6c, 6d, 6e, 6f) when the piece of equipment (6a, 6b, 6c, 6d, 6e, 6f) is in a fourth arrangement, wherein each arrangement is one of a plurality of working positions of the piece of equipment (6a, 6b, 6c, 6d, 6e, 6f), and
determining a crossing of a third line that extends in the third viewing direction through a predetermined point on the portion of the surface (6b-1, 6c-1, 6e-1) of the piece of equipment (6a, 6b, 6c, 6d, 6e, 6f) and a fourth line that extends in the fourth viewing direction through the predetermined point on the portion of the surface (6b-1, 6c-1, 6e-1) of the piece of equipment (6a, 6b, 6c, 6d, 6e, 6f).

9. The method of any of the preceding claims, comprising determining, for each of a plurality of pieces of equipment, the isocenter (6b-3, 6c-3) and/or the pivot point and aligning the pieces of equipment in such a manner that the isocenter (6b-3, 6c-3) and/or the pivot point of one of the pieces of equipment aligns with the isocenter (6b-3, 6c-3) and/or the pivot point of another of the pieces of equipment.

10. The method of any of the preceding claims,
wherein adjusting the alignment of the piece of equipment (6a, 6b, 6c, 6d, 6e, 6f) comprises changing the arrangement of the piece of equipment (6a, 6b, 6c, 6d, 6e, 6f) in such a manner that an improvement of the alignment is expected, and/or
wherein the method comprises, after an arrangement of the piece of equipment (6a, 6b, 6c, 6d, 6e, 6f) has been changed, for example in the course of adjusting the alignment of the piece of equipment (6a, 6b, 6c, 6d, 6e, 6f), determining whether the alignment has improved, in particular, wherein determining whether the alignment has improved comprises determining the viewing direction of the piece of equipment (6a, 6b, 6c, 6d, 6e, 6f) and determining whether the viewing direction is within the predetermined interval around the target viewing direction (6b-5, 6c-5) of the piece of equipment (6a, 6b, 6c, 6d, 6e, 6f) and/or verifying the alignment by an alignment method that does not rely on determining the viewing direction.

11. The method of any of the preceding claims, wherein determining the viewing direction of the piece of equipment (6a, 6b, 6c, 6d, 6e, 6f) comprises analyzing data obtained by a surface camera (6f) observing the portion of the surface (6b-1, 6c-1, 6e-1) of the piece of equipment (6a, 6b, 6c, 6d, 6e, 6f) so as to determine the normal (6b-2, 6c-2, 6e-2) vectors of the surface, for example by means of segmentation of a pointcloud detected by the surface camera (6f), and/or wherein determining the viewing direction of the piece of equipment (6a, 6b, 6c, 6d, 6e, 6f) comprises analyzing data obtained by a camera observing a marker (7a, 7b, 7c, 7e, 7f), arranged on the surface of the piece of equipment (6a, 6b, 6c, 6d, 6e, 6f) in a predetermined arrangement relative to the portion of the surface (6b-1, 6c-1, 6e-1) of the piece of equipment (6a, 6b, 6c, 6d, 6e, 6f).

12. The method of any of the preceding claims,
further comprising overlaying an indication (6b-2', 6c-2') of the viewing direction, in particular one or more of the lines of the preceding claims, as part of a virtual image onto the field of view (5a) of an augmented reality device (5), in case the field of view (5a) comprises the piece of equipment (6a, 6b, 6c, 6d, 6e, 6f), and/or
further comprising the step of overlaying an indication (6b-5', 6c-5') of a target viewing direction (6b-5, 6c-5), in particular one or more target lines, as virtual image onto the field of view (5a) of the augmented reality device (5), in particular at the same time as the indication of the viewing direction, and/or
further comprising providing an indication to a user of an/the augmented reality device (5) when the viewing direction approaches and/or diverges from a/the target viewing direction (6b-5, 6c-5), in particular, prompting the user to initiate verifying and/or adjusting the alignment.

13. The method of any of the preceding claims, further comprising,
accessing alignment data from an alignment and/or an adjustment of the alignment of the piece of equipment (6a, 6b, 6c, 6d, 6e, 6f), the alignment and/or adjustment of an alignment making use of a method not relying on determining the viewing direction, subsequently, determining the viewing direction of the piece of equipment (6a, 6b, 6c, 6d, 6e, 6f),
determining whether there is a discrepancy between the alignment performed prior to determining the viewing direction and an alignment derived from the viewing direction,
if it is determined that there is a discrepancy, performing a calibration of the method used for determining the viewing direction of the piece of equipment (6a, 6b, 6c, 6d, 6e, 6f) so as to reduce the discrepancy, in particular such that there is no discrepancy,
only after determining that there is no discrepancy, performing the step of, upon determining that the viewing direction is outside of the predetermined interval around the target viewing direction (6b-5, 6c-5) of the piece of equipment (6a, 6b, 6c, 6d, 6e, 6f), initiating the adjusting of the alignment of the piece of equipment (6a, 6b, 6c, 6d, 6e, 6f).

14. A medical system (1) comprising
at least one piece of equipment (6a, 6b, 6c, 6d, 6e, 6f), and
a computing system (2) configured to carry out the method of any of the preceding claims.

15. The medical system of claim 14,
comprising one or more imaging devices, in particular a surface camera (6f), configured to obtain image data of the piece of equipment (6a, 6b, 6c, 6d, 6e, 6f), in particular of the portion of the surface (6b-1, 6c-1, 6e-1) of the piece of equipment (6a, 6b, 6c, 6d, 6e, 6f) and/or of a/the marker (7a, 7b, 7c, 7e, 7f) and provide the image data to the computing system (2), wherein the computing system (2) is configured to determine the viewing direction of the piece of equipment (6a, 6b, 6c, 6d, 6e, 6f) based on the image data; and/or
comprising a drive system (4) configured to, in response to receiving a/the control signal to drive the piece of equipment (6a, 6b, 6c, 6d, 6e, 6f) so as to traverse a/the target path, drive the piece of equipment (6a, 6b, 6c, 6d, 6e, 6f); and/or
comprising an/the augmented reality device (5), in particular, wherein at least one of the one or more imaging devices of claim 14 is comprised in or connected to the augmented reality device (5); and/or
wherein the at least one piece of equipment (6a, 6b, 6c, 6d, 6e, 6f) comprises at least one of: a linear accelerator (6a, 6b) for creating a beam, one or more panels for detecting X-ray radiation (6e), one or more cameras (6f), a support unit (6c) for supporting a subject, in particular, a subject to be placed into a/the beam of a/the linear accelerator (6a, 6b); and/or
wherein the computing system (2) is configured to issue control signals to one or more pieces of equipment of the medical system (1) and/or to the drive system (4) and/or the augmented reality device (5), in particular control signals to prompt the one or more imaging devices to obtain data and/or to prompt the augmented reality device (5) to perform the overlaying of the indication of the viewing direction and/or to prompt the drive system (4) to drive a piece of equipment (6a, 6b, 6c, 6d, 6e, 6f); and/or
wherein the medical system (1) comprises a radiation treatment apparatus comprising a treatment beam source and a patient support unit, and wherein the computing system (2) is operably coupled to the radiation treatment apparatus for issuing a control signal to the radiation treatment apparatus for controlling the operation of the treatment beam source and/or the position of the patient support unit.

16. A computer program which, when the program is executed by a computer or when loaded onto a computer, causes the computer to carry out the method steps of the method of any one of claims 1 to 13;
and/or a program storage medium on which the computer program is stored;
and/or a computer comprising at least one processor and a memory and/or the program storage medium, wherein the program is running on the computer or loaded into the memory of the computer;
and/or a signal wave or a digital signal wave, carrying information which represents the program;
and/or a data stream which is representative of the program.

## Patentansprüche

1. Computerimplementiertes Verfahren zur Verwendung beim Ausrichten von Ausrüstungsteilen (6a, 6b, 6c, 6d, 6e, 6f) eines medizinischen Systems (1), wobei das Verfahren umfasst:
Bestimmen (S11), für ein Ausrüstungsteil (6a, 6b, 6c, 6d, 6e, 6f) eines medizinischen Systems (1), einer Blickrichtung des Ausrüstungsteils (6a, 6b, 6c, 6d, 6e, 6f), wobei die Blickrichtung durch eine Normale (6b-2, 6c-2, 6e-2, 6f-2) zu einem zuvor festgelegten Abschnitt der Oberfläche (6b-1, 6c-1, 6e-1) des Ausrüstungsteils (6a, 6b, 6c, 6d, 6e, 6f) beschrieben wird,
Bestimmen (S12), ob die Blickrichtung innerhalb eines zuvor festgelegten Intervalls um eine Zielblickrichtung (6b-5, 6c-5) des Ausrüstungsteils (6a, 6b, 6c, 6d, 6e, 6f) liegt, und
nach dem Bestimmen, dass die Blickrichtung außerhalb des zuvor festgelegten Intervalls um die Zielblickrichtung (6b-5, 6c-5) des Ausrüstungsteils (6a, 6b, 6c, 6d, 6e, 6f) liegt, Initiieren (S13) einer Justierung der Ausrichtung des Ausrüstungsteils (6a, 6b, 6c, 6d, 6e, 6f).

2. Verfahren nach Anspruch 1, des Weiteren umfassend das Verfolgen der Ausrichtung des Ausrüstungsteils (6a, 6b, 6c, 6d, 6e, 6f), wobei das Verfolgen das wiederholte Bestimmen, zum Beispiel an zuvor festgelegten Zeitpunkten und/oder Zeitintervallen oder kontinuierlich, umfasst, ob die Blickrichtung innerhalb des zuvor festgelegten Intervalls um die Zielblickrichtung (6b-5, 6c-5) des Ausrüstungsteils (6a, 6b, 6c, 6d, 6e, 6f) liegt.

3. Verfahren nach Anspruch 1 oder 2, wobei das Verfahren, insbesondere das Bestimmen, ob die Blickrichtung innerhalb des zuvor festgelegten Intervalls liegt, umfasst:
Bestimmen, ob eine Linie, die sich in der Blickrichtung durch einen zuvor festgelegten Punkt auf dem Abschnitt der Oberfläche (6b-1, 6c-1, 6e-1) des Ausrüstungsteils (6a, 6b, 6c, 6d, 6e, 6f) erstreckt, auch durch mindestens einen weiteren zuvor festgelegten Punkt erstreckt, wobei der weitere zuvor festgelegte Punkt ein zuvor festgelegter Punkt auf einer Oberfläche eines anderen Ausrüstungsteils (6a, 6b, 6c, 6d, 6e, 6f) und/oder ein Raum-Isozentrum (6b-3, 6c-3) und/oder ein Isozentrum (6b-3, 6c-3) eines anderen Ausrüstungsteils (6a, 6b, 6c, 6d, 6e, 6f) sein kann, und/oder
Bestimmen, ob eine/die Linie, die sich in der Blickrichtung durch einen/den zuvor festgelegten Punkt auf dem Abschnitt der Oberfläche (6b-1, 6c-1, 6e-1) des Ausrüstungsteils (6a, 6b, 6c, 6d, 6e, 6f) erstreckt, auch durch mindestens eine zuvor festgelegte Region erstreckt, wobei die zuvor festgelegte Region ein zuvor festgelegter Abschnitt einer Oberfläche eines anderen Ausrüstungsteils (6a, 6b, 6c, 6d, 6e, 6f) und/oder eine zuvor festgelegter Region, in der sich ein/das Raum-Isozentrum (6b-3, 6c-3) und/oder ein/das Isozentrum (6b-3, 6c-3) eines anderen Ausrüstungsteils (6a, 6b, 6c, 6d, 6e, 6f) befindet, sein kann.

4. Verfahren nach Anspruch 3, wobei der zuvor festgelegte Punkt und/oder die zuvor festgelegte Region in einer zuvor festgelegten Anordnung relativ zu mindestens einer Markierung (7a, 7b, 7c, 7e, 7f) angeordnet sind und das Bestimmen, ob sich die Linie durch den zuvor festgelegten Punkt und/oder die zuvor festgelegte Region erstreckt, umfasst:
Bestimmen der relativen Anordnung der Linie relativ zu der Markierung (7a, 7b, 7c, 7e, 7f), und
Ableiten der Anordnung des zuvor festgelegten Punktes und/oder der zuvor festgelegten Region relativ zu der Linie aus der relativen Anordnung der Linie relativ zu der Markierung (7a, 7b, 7c, 7e, 7f) und aus der zuvor festgelegten Anordnung des zuvor festgelegten Punktes und/oder der zuvor festgelegten Region relativ zu der Markierung (7a, 7b, 7c, 7e, 7f).

5. Verfahren nach einem der vorangehenden Ansprüche, des Weiteren umfassend:
Bestimmen eines Schwenkpunktes eines/des Ausrüstungsteils (6a, 6b, 6c, 6d, 6e, 6f) des medizinischen Systems (1),
wobei das Bestimmen des Schwenkpunktes umfasst:
Bestimmen einer ersten Blickrichtung des Ausrüstungsteils (6a, 6b, 6c, 6d, 6e, 6f), wenn sich das Ausrüstungsteil (6a, 6b, 6c, 6d, 6e, 6f) in einer ersten Anordnung befindet, und einer zweiten Blickrichtung des Ausrüstungsteils (6a, 6b, 6c, 6d, 6e, 6f), wenn das Ausrüstungsteil (6a, 6b, 6c, 6d, 6e, 6f) so geschwenkt wurde, dass es sich in einer zweiten Anordnung befindet,
Bestimmen eines Kreuzungspunktes einer ersten Linie, die sich in der ersten Blickrichtung durch einen zuvor festgelegten Punkt auf dem Abschnitt der Oberfläche (6b-1, 6c-1, 6e-1) des Ausrüstungsteils (6a, 6b, 6c, 6d, 6e, 6f) erstreckt, und einer zweiten Linie, die sich in der zweiten Blickrichtung durch den zuvor festgelegten Punkt auf dem Abschnitt der Oberfläche (6b-1, 6c-1, 6e-1) des Ausrüstungsteils (6a, 6b, 6c, 6d, 6e, 6f) erstreckt, und
Identifizieren des Kreuzungspunktes als den Schwenkpunkt.

6. Verfahren nach einem der vorangehenden Ansprüche, des Weiteren umfassend:
Bestimmen, ob ein tatsächlicher Pfad, der durch ein/das Ausrüstungsteil (6a, 6b, 6c, 6d, 6e, 6f) des medizinischen Systems (1) durchlaufen wird, einem Zielpfad entspricht, der durch das Ausrüstungsteil durchlaufen werden soll,
wobei das Bestimmen, ob der tatsächliche Pfad dem Zielpfad entspricht, umfasst:
Senden eines Steuersignals an ein Antriebssystem (4), wobei das Steuersignal das Antriebssystem (4) anweist, das Ausrüstungsteil (6a, 6b, 6c, 6d, 6e, 6f) so anzutreiben, dass es den Zielpfad durchläuft,
Bestimmen der Blickrichtungen des Ausrüstungsteils (6a, 6b, 6c, 6d, 6e, 6f) bei mehreren Anordnungen des Ausrüstungsteils (6a, 6b, 6c, 6d, 6e, 6f) entlang eines tatsächlichen Pfades, den das Ausrüstungsteil (6a, 6b, 6c, 6d, 6e, 6f), angetrieben durch das Antriebssystem (4) in Reaktion auf das Empfangen des Steuersignals, durchläuft,
Bestimmen, für jede der mehreren Anordnungen, ob eine Linie, die sich entlang der Blickrichtung durch einen zuvor festgelegten Punkt auf der Oberfläche des Ausrüstungsteils (6a, 6b, 6c, 6d, 6e, 6f) erstreckt, auch durch einen zuvor festgelegten Punkt und/oder eine zuvor festgelegte Region erstreckt, insbesondere, ob sie eine Linie schneidet, die sich entlang der Blickrichtung durch den zuvor festgelegten Punkt auf der Oberfläche des Ausrüstungsteils (6a, 6b, 6c, 6d, 6e, 6f) einer oder mehrerer anderer Anordnungen erstreckt,
falls dies der Fall ist, Bestimmen, dass der tatsächliche Pfad, der durch das Ausrüstungsteil (6a, 6b, 6c, 6d, 6e, 6f) durchlaufen wird, dem Zielpfad entspricht, der durch das Ausrüstungsteil (6a, 6b, 6c, 6d, 6e, 6f) durchlaufen werden soll, und
anderenfalls Bestimmen, dass eine Abweichung zwischen dem tatsächlichen Pfad, der durch das Ausrüstungsteil (6a, 6b, 6c, 6d, 6e, 6f) durchlaufen wird, und dem Zielpfad, der durch das Ausrüstungsteil (6a, 6b, 6c, 6d, 6e, 6f) durchlaufen werden soll, besteht.

7. Verfahren nach Anspruch 6, des Weiteren umfassend, wenn bestimmt wird, dass eine Abweichung zwischen dem tatsächlichen Pfad, der durch das Ausrüstungsteil (6a, 6b, 6c, 6d, 6e, 6f) durchlaufen wird, und dem Zielpfad, der durch das Ausrüstungsteil (6a, 6b, 6c, 6d, 6e, 6f) durchlaufen werden soll, besteht, Bestimmen weiterer Informationen bezüglich der Abweichung, zum Beispiel durch Bestimmen der Blickrichtungen für zusätzliche Anordnungen und/oder durch Bestimmen der Abweichung der tatsächlichen Linie, die sich entlang der Blickrichtung erstreckt, von der Ziellinie, die sich entlang der Blickrichtung erstreckt, für jede der Anordnungen.

8. Verfahren nach einem der vorangehenden Ansprüche, des Weiteren umfassend:
Bestimmen, für ein/das Ausrüstungsteil (6a, 6b, 6c, 6d, 6e, 6f) des medizinischen Systems (1), eines Isozentrums (6b-3, 6c-3) des Ausrüstungsteils (6a, 6b, 6c, 6d, 6e, 6f),
wobei das Bestimmen des Isozentrums (6b-3, 6c-3) umfasst:
Bestimmen einer dritten Blickrichtung des Ausrüstungsteils (6a, 6b, 6c, 6d, 6e, 6f), wenn sich das Ausrüstungsteil (6a, 6b, 6c, 6d, 6e, 6f) in einer dritten Anordnung befindet, und einer vierten Blickrichtung des Ausrüstungsteils (6a, 6b, 6c, 6d, 6e, 6f), wenn sich das Ausrüstungsteil (6a, 6b, 6c, 6d, 6e, 6f) in einer vierten Anordnung befindet, wobei jede Anordnung eine von mehreren Arbeitspositionen des Ausrüstungsteils (6a, 6b, 6c, 6d, 6e, 6f) ist, und
Bestimmen eines Schnittpunktes einer dritten Linie, die sich in der dritten Blickrichtung durch einen zuvor festgelegten Punkt auf dem Abschnitt der Oberfläche (6b-1, 6c-1, 6e-1) des Ausrüstungsteils (6a, 6b, 6c, 6d, 6e, 6f) erstreckt, und einer vierten Linie, die sich in der vierten Blickrichtung durch den zuvor festgelegten Punkt auf dem Abschnitt der Oberfläche (6b-1, 6c-1, 6e-1) des Ausrüstungsteils (6a, 6b, 6c, 6d, 6e, 6f) erstreckt.

9. Verfahren nach einem der vorangehenden Ansprüche, umfassend das Bestimmen, für jedes von mehreren Ausrüstungsteilen, des Isozentrums (6b-3, 6c-3) und/oder des Schwenkpunktes und Ausrichten der Ausrüstungsteile in einer solchen Weise, dass das Isozentrum (6b-3, 6c-3) und/oder der Schwenkpunkt eines der Ausrüstungsteile auf das Isozentrum (6b-3, 6c-3) und/oder den Schwenkpunkt eines anderen der Ausrüstungsteile ausgerichtet ist.

10. Verfahren nach einem der vorangehenden Ansprüche,
wobei das Justieren der Ausrichtung des Ausrüstungsteils (6a, 6b, 6c, 6d, 6e, 6f) das Ändern der Anordnung des Ausrüstungsteils (6a, 6b, 6c, 6d, 6e, 6f) in einer solchen Weise umfasst, dass eine Verbesserung der Ausrichtung erwartet wird, und/oder
wobei das Verfahren, nachdem eine Anordnung des Ausrüstungsteils (6a, 6b, 6c, 6d, 6e, 6f) geändert wurde, zum Beispiel im Zuge des Justierens der Ausrichtung des Ausrüstungsteils (6a, 6b, 6c, 6d, 6e, 6f), das Bestimmen umfasst, ob sich die Ausrichtung verbessert hat, wobei insbesondere das Bestimmen, ob sich die Ausrichtung verbessert hat, das Bestimmen der Blickrichtung des Ausrüstungsteils (6a, 6b, 6c, 6d, 6e, 6f) und das Bestimmen, ob die Blickrichtung innerhalb des zuvor festgelegten Intervalls um die Zielblickrichtung (6b-5, 6c-5) des Ausrüstungsteils (6a, 6b, 6c, 6d, 6e, 6f) liegt, und/oder das Verifizieren der Ausrichtung durch ein Ausrichtungsverfahren, das nicht auf dem Bestimmen der Blickrichtung beruht, umfasst.

11. Verfahren nach einem der vorangehenden Ansprüche, wobei das Bestimmen der Blickrichtung des Ausrüstungsteils (6a, 6b, 6c, 6d, 6e, 6f) das Analysieren von Daten umfasst, die durch eine Oberflächenkamera (6f) erhalten werden, die den Abschnitt der Oberfläche (6b-1, 6c-1, 6e-1) des Ausrüstungsteils (6a, 6b, 6c, 6d, 6e, 6f) beobachtet, um die Vektoren der Normalen (6b-2, 6c-2, 6e-2) der Oberfläche, zum Beispiel mittels Segmentierung einer durch die Oberflächenkamera (6f) erfassten Punktwolke, zu bestimmen, und/oder wobei das Bestimmen der Blickrichtung des Ausrüstungsteils (6a, 6b, 6c, 6d, 6e, 6f) das Analysieren von Daten umfasst, die durch eine Kamera erhalten werden, die eine Markierung (7a, 7b, 7c, 7e, 7f) beobachtet, die auf der Oberfläche des Ausrüstungsteils (6a, 6b, 6c, 6d, 6e, 6f) in einer zuvor festgelegten Anordnung relativ zu dem Abschnitt der Oberfläche (6b-1, 6c-1, 6e-1) des Ausrüstungsteils (6a, 6b, 6c, 6d, 6e, 6f) angeordnet ist.

12. Verfahren nach einem der vorangehenden Ansprüche,
des Weiteren umfassend das Überlagern eines Hinweises (6b-2', 6c-2') bezüglich der Blickrichtung, insbesondere einer oder mehrerer der Linien der vorangehenden Ansprüche, als Teil eines virtuellen Bildes auf das Sichtfeld (5a) einer Augmented-Reality-Vorrichtung (5), falls das Sichtfeld (5a) das Ausrüstungsteil (6a, 6b, 6c, 6d, 6e, 6f) umfasst, und/oder
des Weiteren umfassend den Schritt des Überlagerns eines Hinweises (6b-5', 6c-5') bezüglich einer Zielblickrichtung (6b-5, 6c-5), insbesondere einer oder mehrerer Ziellinien, als virtuelles Bild auf das Sichtfeld (5a) der Augmented-Reality-Vorrichtung (5), insbesondere gleichzeitig mit dem Hinweis bezüglich der Blickrichtung, und/oder
des Weiteren umfassend das Bereitstellen eines Hinweises an einen Benutzer einer/der Augmented-Reality-Vorrichtung (5), wenn sich die Blickrichtung einer/der Zielblickrichtung (6b-5, 6c-5) nähert und/oder von dieser abweicht, insbesondere Auffordern des Benutzers, das Verifizieren und/oder Justieren der Ausrichtung zu initiieren.

13. Verfahren nach einem der vorangehenden Ansprüche, des Weiteren umfassend:
Zugreifen auf Ausrichtungsdaten von einer Ausrichtung und/oder einer Justierung der Ausrichtung des Ausrüstungsteils (6a, 6b, 6c, 6d, 6e, 6f), wobei für die Ausrichtung und/oder Justierung einer Ausrichtung ein Verfahren verwendet wird, das nicht auf dem Bestimmen der Blickrichtung beruht,
anschließendes Bestimmen der Blickrichtung des Ausrüstungsteils (6a, 6b, 6c, 6d, 6e, 6f),
Bestimmen, ob eine Diskrepanz zwischen der Ausrichtung, die vor dem Bestimmen der Blickrichtung durchgeführt wurde, und einer Ausrichtung, die von der Blickrichtung abgeleitet wurde, besteht,
wenn bestimmt wird, dass eine Diskrepanz besteht, Durchführen einer Kalibrierung des Verfahrens, das zum Bestimmen der Blickrichtung des Ausrüstungsteils (6a, 6b, 6c, 6d, 6e, 6f) verwendet wird, um die Diskrepanz zu verringern, insbesondere so, dass keine Diskrepanz mehr besteht,
erst nach dem Bestimmen, dass keine Diskrepanz besteht, Durchführen - nach dem Bestimmen, dass die Blickrichtung außerhalb des zuvor festgelegten Intervalls um die Zielblickrichtung (6b-5, 6c-5) des Ausrüstungsteils (6a, 6b, 6c, 6d, 6e, 6f) liegt - des Schrittes des Initiierens des Justierens der Ausrichtung des Ausrüstungsteils (6a, 6b, 6c, 6d, 6e, 6f).

14. Medizinisches System (1), umfassend:
mindestens ein Ausrüstungsteil (6a, 6b, 6c, 6d, 6e, 6f), und
ein Computersystem (2), das dazu eingerichtet ist, das Verfahren nach einem der vorangehenden Ansprüche auszuführen.

15. Medizinisches System nach Anspruch 14,
umfassend eine oder mehrere Bildgebungsvorrichtungen, insbesondere eine Oberflächenkamera (6f), die dazu eingerichtet sind, Bilddaten des Ausrüstungsteils (6a, 6b, 6c, 6d, 6e, 6f), insbesondere des Abschnitts der Oberfläche (6b-1, 6c-1, 6e-1) des Ausrüstungsteils (6a, 6b, 6c, 6d, 6e, 6f) und/oder einer/der Markierung (7a, 7b, 7c, 7e, 7f), zu erhalten und die Bilddaten an das Computersystem (2) zu übermitteln, wobei das Computersystem (2) dazu eingerichtet ist, die Blickrichtung des Ausrüstungsteils (6a, 6b, 6c, 6d, 6e, 6f) auf der Grundlage der Bilddaten zu bestimmen; und/oder
umfassend ein Antriebssystem (4), das dazu eingerichtet ist, in Reaktion auf das Empfangen eines/des Steuersignals zum Antreiben des Ausrüstungsteils (6a, 6b, 6c, 6d, 6e, 6f) so, dass es einen/den Zielpfad durchläuft, das Ausrüstungsteil (6a, 6b, 6c, 6d, 6e, 6f) anzutreiben; und/oder
umfassend eine/die Augmented-Reality-Vorrichtung (5), wobei insbesondere mindestens eine der einen oder der mehreren Bildgebungsvorrichtungen nach Anspruch 14 in der Augmented-Reality-Vorrichtung (5) enthalten oder mit dieser verbunden ist; und/oder
wobei das mindestens eine Ausrüstungsteil (6a, 6b, 6c, 6d, 6e, 6f) mindestens eines von Folgendem umfasst: einen Linearbeschleuniger (6a, 6b) zum Erzeugen eines Strahls, ein oder mehrere Paneele zum Detektieren von Röntgenstrahlung (6e), eine oder mehrere Kameras (6f), eine Stützeinheit (6c) zum Stützen einer Person, insbesondere einer Person, die in einen/den Strahl eines/des Linearbeschleunigers (6a, 6b) platziert werden soll; und/oder
wobei das Computersystem (2) dazu eingerichtet ist, Steuersignale an ein oder mehrere Ausrüstungsteile des medizinischen Systems (1) und/oder an das Antriebssystem (4) und/oder die Augmented-Reality-Vorrichtung (5) auszugeben, insbesondere Steuersignale, um die eine oder die mehreren Bildgebungsvorrichtungen zu veranlassen, Daten zu erhalten, und/oder um die Augmented-Reality-Vorrichtung (5) zu veranlassen, das Überlagern des Hinweises bezüglich der Blickrichtung durchzuführen, und/oder um das Antriebssystem (4) zu veranlassen, ein Ausrüstungsteil (6a, 6b, 6c, 6d, 6e, 6f) anzutreiben; und/oder
wobei das medizinische System (1) eine Strahlungsbehandlungsvorrichtung umfasst, die eine Behandlungsstrahlquelle und eine Patientenauflageeinheit umfasst, und wobei das Computersystem (2) mit der Strahlungsbehandlungsvorrichtung wirkgekoppelt ist, um ein Steuersignal an die Strahlungsbehandlungsvorrichtung auszugeben, um den Betrieb der Behandlungsstrahlquelle und/oder die Position der Patientenauflageeinheit zu steuern.

16. Computerprogramm, das, wenn das Programm durch einen Computer ausgeführt oder in einen Computer geladen wird, den Computer veranlasst, die Verfahrensschritte des Verfahrens nach einem der Ansprüche 1 bis 13 auszuführen;
und/oder ein Programmspeichermedium, auf dem das Computerprogramm gespeichert ist;
und/oder einen Computer, der mindestens einen Prozessor und einen Speicher und/oder das Programmspeichermedium umfasst, wobei das Programm auf dem Computer läuft oder in den Speicher des Computers geladen ist;
und/oder eine Signalwelle oder eine digitale Signalwelle, die Informationen transportiert, die das Programm repräsentieren;
und/oder einen Datenstrom, der für das Programm repräsentativ ist.

## Revendications

1. Procédé mis en œuvre par ordinateur destiné à être utilisé pour l'alignement d'éléments d'équipement (6a, 6b, 6c, 6d, 6e, 6f) d'un système médical (1), le procédé comprenant :
la détermination (S11), pour un élément d'équipement (6a, 6b, 6c, 6d, 6e, 6f) d'un système médical (1), d'une direction de visée de l'élément d'équipement (6a, 6b, 6c, 6d, 6e, 6f), la direction de visée étant décrite par une normale (6b-2, 6c-2, 6e-2, 6f-2) à une partie prédéterminée de la surface (6b-1, 6c-1, 6e-1) de l'élément d'équipement (6a, 6b, 6c, 6d, 6e, 6f),
le fait de déterminer (S12) si la direction de visée se situe dans un intervalle prédéterminé autour d'une direction de visée cible (6b-5, 6c-5) de l'élément d'équipement (6a, 6b, 6c, 6d, 6e, 6f), et
lorsqu'il est déterminé que la direction de visée se situe en dehors de l'intervalle prédéterminé autour de la direction de visée cible (6b-5, 6c-5) de l'élément d'équipement (6a, 6b, 6c, 6d, 6e, 6f), l'initiation (S13) d'un ajustement de l'alignement de l'élément d'équipement (6a, 6b, 6c, 6d, 6e, 6f).

2. Procédé selon la revendication 1, comprenant en outre le suivi de l'alignement de l'élément d'équipement (6a, 6b, 6c, 6d, 6e, 6f), le suivi comprenant le fait de déterminer de manière répétée, par exemple à des moments et/ou intervalles de temps prédéterminés ou en continu, si la direction de visée se situe dans l'intervalle prédéterminé autour de la direction de visée cible (6b-5, 6c-5) de l'élément d'équipement (6a, 6b, 6c, 6d, 6e, 6f).

3. Procédé selon la revendication 1 ou 2, dans lequel le procédé, en particulier le fait de déterminer si la direction de visée se situe dans l'intervalle prédéterminé, comprend :
le fait de déterminer si une ligne s'étendant dans la direction de visée à travers un point prédéterminé sur la partie de la surface (6b-1,6c-1,6e-1) de l'élément d'équipement (6a, 6b, 6c, 6d, 6e, 6f) s'étend également à travers au moins un autre point prédéterminé, dans lequel l'autre point prédéterminé peut être un point prédéterminé sur une surface d'un autre élément d'équipement (6a, 6b, 6c, 6d, 6e, 6f) et/ou un isocentre de pièce (6b-3,6c-3) et/ou un isocentre (6b-3, 6c-3) d'un autre élément d'équipement (6a, 6b, 6c, 6d, 6e, 6f), et/ou
le fait de déterminer si une/la ligne s'étendant dans la direction de visée à travers un/le point prédéterminé sur la partie de la surface (6b-1, 6c-1, 6e-1) de l'élément d'équipement (6a, 6b, 6c, 6d, 6e, 6f) s'étend également à travers au moins une région prédéterminée, dans lequel la région prédéterminée peut être une partie prédéterminée d'une surface d'un autre élément d'équipement (6a, 6b, 6c, 6d, 6e, 6f) et/ou une région prédéterminée dans laquelle se trouve un/l'isocentre de pièce (6b-3, 6c-3) et/ou un/l'isocentre (6b-3, 6c-3) d'un autre élément d'équipement (6a, 6b, 6c, 6d, 6e, 6f).

4. Procédé selon la revendication 3, dans lequel le point prédéterminé et/ou la région prédéterminée sont disposés dans une disposition prédéterminée par rapport à au moins un marqueur (7a, 7b, 7c, 7e, 7f) et le fait de déterminer si la ligne s'étend à travers le point prédéterminé et/ou la région prédéterminée comprend :
la détermination de la disposition relative de la ligne par rapport au marqueur (7a, 7b, 7c, 7e, 7f), et
la déduction de la disposition du point prédéterminé et/ou de la région prédéterminée par rapport à la ligne à partir de la disposition relative de la ligne par rapport au marqueur (7a, 7b, 7c, 7e, 7f) et de la disposition prédéterminée du point prédéterminé et/ou de la région prédéterminée par rapport au marqueur (7a, 7b, 7c, 7e, 7f).

5. Procédé selon l'une quelconque des revendications précédentes, comprenant en outre
la détermination d'un point de pivot d'un/de l'élément d'équipement (6a, 6b, 6c, 6d, 6e, 6f) du système médical (1),
dans lequel la détermination du point de pivot comprend :
la détermination d'une première direction de visée de l'élément d'équipement (6a, 6b, 6c, 6d, 6e, 6f) lorsque l'élément d'équipement (6a, 6b, 6c, 6d, 6e, 6f) est dans une première disposition et d'une deuxième direction de visée de l'élément d'équipement (6a, 6b, 6c, 6d, 6e, 6f) lorsque l'élément d'équipement (6a, 6b, 6c, 6d, 6e, 6f) a été pivoté de manière à être dans une seconde disposition,
la détermination d'un point de croisement entre une première ligne s'étendant dans la première direction de visée à travers par un point prédéterminé sur la partie de la surface (6b-1, 6c-1, 6e-1) de l'élément d'équipement (6a, 6b, 6c, 6d, 6e, 6f) et une seconde ligne s'étendant dans la deuxième direction de visée à travers le point prédéterminé sur la partie de la surface (6b-1, 6c-1, 6e-1) de l'élément d'équipement (6a, 6b, 6c, 6d, 6e, 6f), et
l'identification du point de croisement comme étant le point de pivot.

6. Procédé selon l'une quelconque des revendications précédentes, comprenant en outre
le fait de déterminer si une trajectoire réelle parcourue par un/l'élément d'équipement (6a, 6b, 6c, 6d, 6e, 6f) du système médical (1) correspond à une trajectoire cible à parcourir par l'élément d'équipement,
dans lequel le fait de déterminer si la trajectoire réelle correspond à la trajectoire cible comprend :
l'envoi d'un signal de commande à un système d'entraînement (4), le signal de commande ordonnant au système d'entraînement (4) d'entraîner l'élément d'équipement (6a, 6b, 6c, 6d, 6e, 6f) de manière à parcourir la trajectoire cible,
la détermination des directions de visée de l'élément d'équipement (6a, 6b, 6c, 6d, 6e, 6f) à de multiples dispositions de l'élément d'équipement (6a, 6b, 6c, 6d, 6e, 6f) le long d'une trajectoire réelle que l'élément d'équipement (6a, 6b, 6c, 6d, 6e, 6f), entraîné par le système d'entraînement (4) en réponse à la réception d'un signal de commande, parcourt,
le fait de déterminer, pour chacune des multiples dispositions, si une ligne s'étendant le long de la direction de visée à travers un point prédéterminé sur la surface de l'élément d'équipement (6a, 6b, 6c, 6d, 6e, 6f) s'étend également à travers un point et/ou une région prédéterminés, en particulier si elle croise une ligne s'étendant le long de la direction de visée à travers le point prédéterminé sur la surface de l'élément d'équipement (6a, 6b, 6c, 6d, 6e, 6f) d'une ou de plusieurs autres dispositions,
si tel est le cas, le fait de déterminer que la trajectoire réelle parcourue par l'élément d'équipement (6a, 6b, 6c, 6d, 6e, 6f) correspond à la trajectoire cible à parcourir par l'élément d'équipement (6a, 6b, 6c, 6d, 6e, 6f), et
dans le cas contraire, la détermination qu'il existe un écart entre la trajectoire réelle parcourue par l'élément d'équipement (6a, 6b, 6c, 6d, 6e, 6f) et la trajectoire cible à parcourir par l'élément d'équipement (6a, 6b, 6c, 6d, 6e, 6f).

7. Procédé selon la revendication 6, comprenant en outre, lorsqu'il est déterminé qu'il existe un écart entre la trajectoire réelle parcourue par l'élément d'équipement (6a, 6b, 6c, 6d, 6e, 6f) et la trajectoire cible à parcourir par l'élément d'équipement (6a, 6b, 6c, 6d, 6e, 6f), la détermination d'autres informations concernant l'écart, par exemple en déterminant les directions de visée pour des dispositions supplémentaires et/ou en déterminant l'écart de la ligne réelle s'étendant le long de la direction de visée par rapport à la ligne cible s'étendant le long de la direction de visée pour chacune des dispositions.

8. Procédé selon l'une quelconque des revendications précédentes, comprenant en outre
la détermination, pour un/l'élément d'équipement (6a, 6b, 6c, 6d, 6e, 6f) du système médical (1), d'un isocentre (6b-3, 6c-3) de l'élément d'équipement (6a, 6b, 6c, 6d, 6e, 6f),
dans lequel la détermination de l'isocentre (6b-3, 6c-3) comprend :
la détermination d'une troisième direction de visée de l'élément d'équipement (6a, 6b, 6c, 6d, 6e, 6f) lorsque l'élément d'équipement (6a, 6b, 6c, 6d, 6e, 6f) est dans une troisième disposition et d'une quatrième direction de visée de l'élément d'équipement (6a, 6b, 6c, 6d, 6e, 6f) lorsque l'élément d'équipement (6a, 6b, 6c, 6d, 6e, 6f) est dans une quatrième disposition, dans lequel chaque disposition est l'une d'une pluralité de positions de travail de l'élément d'équipement (6a, 6b, 6c, 6d, 6e, 6f), et
la détermination d'un croisement d'une troisième ligne qui s'étend dans la troisième direction de visée à travers un point prédéterminé sur la partie de la surface (6b-1, 6c-1, 6e-1) de l'élément d'équipement (6a, 6b, 6c, 6d, 6e, 6f) et d'une quatrième ligne qui s'étend dans la quatrième direction de visée à travers le point prédéterminé sur la partie de la surface (6b-1, 6c-1, 6e-1) de l'élément d'équipement (6a, 6b, 6c, 6d, 6e, 6f).

9. Procédé selon l'une quelconque des revendications précédentes, comprenant la détermination, pour chacun d'une pluralité d'éléments d'équipements, de l'isocentre (6b-3, 6c-3) et/ou du point de pivot et l'alignement des éléments d'équipements de manière à ce que l'isocentre (6b-3, 6c-3) et/ou le point de pivot de l'un des éléments d'équipements soit aligné avec l'isocentre (6b-3, 6c-3) et/ou le point de pivot d'un autre des éléments d'équipements.

10. Procédé selon l'une quelconque des revendications précédentes,
dans lequel l'ajustement de l'alignement de l'élément d'équipement (6a, 6b, 6c, 6d, 6e, 6f) comprend la modification de la disposition de l'élément d'équipement (6a, 6b, 6c, 6d, 6e, 6f) de telle sorte qu'une amélioration de l'alignement est attendue, et/ou
dans lequel le procédé comprend, après qu'une disposition de l'élément d'équipement (6a, 6b, 6c, 6d, 6e, 6f) a été modifiée, par exemple au cours de l'ajustement de l'alignement de l'élément d'équipement (6a, 6b, 6c, 6d, 6e, 6f), le fait de déterminer si l'alignement a été amélioré, en particulier, dans lequel le fait de déterminer si l'alignement a été amélioré comprend le fait de déterminer la direction de visée de l'élément d'équipement (6a, 6b, 6c, 6d, 6e, 6f) et le fait de déterminer si la direction de visée se situe dans l'intervalle prédéterminé autour de la direction de visée cible (6b-5, 6c-5) de l'élément d'équipement (6a, 6b, 6c, 6d, 6e, 6f) et/ou le fait de vérifier l'alignement par une méthode d'alignement qui ne repose pas sur la détermination de la direction de visée.

11. Procédé selon l'une quelconque des revendications précédentes, dans lequel la détermination de la direction de visée de l'élément d'équipement (6a, 6b, 6c, 6d, 6e, 6f) comprend l'analyse de données obtenues par une caméra de surface (6f) observant la partie de la surface (6b-1,6c-1,6e-1) de l'élément d'équipement (6a, 6b, 6c, 6d, 6e, 6f) de manière à déterminer les vecteurs normaux (6b-2, 6c- 2, 6e-2) de la surface, par exemple au moyen de la segmentation d'un nuage de points détecté par la caméra de surface (6f), et/ou dans lequel la détermination de la direction de visée de l'élément d'équipement (6a, 6b, 6c, 6d, 6e, 6f) comprend l'analyse de données obtenues par une caméra observant un marqueur (7a, 7b, 7c, 7e, 7f), disposé sur la surface de l'élément d'équipement (6a, 6b, 6c, 6d, 6e, 6f) dans une disposition prédéterminée par rapport à la partie de la surface (6b-1,6c-1,6e-1) de l'élément d'équipement (6a, 6b, 6c, 6d, 6e, 6f).

12. Procédé selon l'une quelconque des revendications précédentes,
comprenant en outre la superposition d'une indication (6b-2', 6c-2') de la direction de visée, en particulier une ou plusieurs des lignes des revendications précédentes, en tant que partie d'une image virtuelle sur le champ de vision (5a) d'un dispositif de réalité augmentée (5), dans le cas où le champ de vision (5a) comprend l'élément d'équipement (6a, 6b, 6c, 6d, 6e, 6f), et/ou
comprenant en outre l'étape consistant à superposer une indication (6b-5', 6c-5') d'une direction de visée cible (6b-5, 6c-5), en particulier une ou plusieurs lignes de visée, en tant qu'image virtuelle sur le champ de vision (5a) du dispositif de réalité augmentée (5), en particulier en même temps que l'indication de la direction de visée, et/ou
comprenant en outre la fourniture d'une indication à un utilisateur d'un/du dispositif de réalité augmentée (5) lorsque la direction de visée s'approche et/ou s'écarte d'une/de la direction de visée cible (6b-5, 6c-5), en particulier en invitant l'utilisateur à commencer à vérifier et/ou à ajuster l'alignement.

13. Procédé selon l'une quelconque des revendications précédentes, comprenant en outre,
l'accès à des données d'alignement provenant d'un alignement et/ou d'un ajustement de l'alignement de l'élément d'équipement (6a, 6b, 6c, 6d, 6e, 6f), l'alignement et/ou l'ajustement d'un alignement faisant appel à une méthode ne reposant pas sur la détermination de la direction de visée,
ensuite, la détermination de la direction de visée de l'élément d'équipement (6a, 6b, 6c, 6d, 6e, 6f),
le fait de déterminer s'il existe une divergence entre l'alignement effectué avant la détermination de la direction de visée et un alignement obtenu à partir de la direction de visée,
s'il est déterminé qu'il y a une divergence, l'effection d'un étalonnage de la méthode utilisée pour déterminer la direction de visée de l'élément d'équipement (6a, 6b, 6c, 6d, 6e, 6f) de manière à réduire la divergence, en particulier de manière à ce qu'il n'y ait pas de divergence,
seulement après avoir déterminé qu'il n'y a pas de divergence, l'effection de l'étape consistant, après avoir déterminé que la direction de visée se trouve en dehors de l'intervalle prédéterminé autour de la direction de visée cible (6b-5, 6c-5) de l'élément d'équipement (6a, 6b, 6c, 6d, 6e, 6f), à initier l'ajustement de l'alignement de l'élément d'équipement (6a, 6b, 6c, 6d, 6e, 6f).

14. Système médical (1) comprenant
au moins un élément d'équipement (6a, 6b, 6c, 6d, 6e, 6f), et
un système informatique (2) configuré pour mettre en œuvre le procédé selon l'une quelconque des revendications précédentes.

15. Système médical selon la revendication 14,
comprenant un ou plusieurs dispositifs d'imagerie, en particulier une caméra de surface (6f), configurés pour obtenir des données d'image de l'élément d'équipement (6a, 6b, 6c, 6d, 6e, 6f), en particulier de la partie de la surface (6b-1,6c-1,6e-1) de l'élément d'équipement (6a, 6b, 6c, 6d, 6e, 6f) et/ou d'un/du marqueur (7a, 7b, 7c, 7e, 7f) et fournir les données d'image au système informatique (2), dans lequel le système informatique (2) est configuré pour déterminer la direction de visée de l'élément d'équipement (6a, 6b, 6c, 6d, 6e, 6f) sur la base des données d'image ; et/ou
comprenant un système d'entraînement (4) configuré pour, en réponse à la réception d'un/du signal de commande pour entraîner l'élément d'équipement (6a, 6b, 6c, 6d, 6e, 6f) de manière à parcourir une/la trajectoire cible, entraîner l'élément d'équipement (6a, 6b, 6c, 6d, 6e, 6f) ; et/ou
comprenant un/le dispositif de réalité augmentée (5), en particulier, dans lequel au moins un du ou des dispositifs d'imagerie selon la revendication 14 est compris dans le dispositif de réalité augmentée (5) ou connecté à celui-ci ; et/ou
dans lequel l'au moins un élément d'équipement (6a, 6b, 6c, 6d, 6e, 6f) comprend au moins l'un parmi : un accélérateur linéaire (6a, 6b) pour créer un faisceau, un ou plusieurs panneaux pour détecter les rayons X (6e), une ou plusieurs caméras (6f), une unité de support (6c) pour soutenir un sujet, en particulier un sujet à placer dans un/le faisceau d'un/de l'accélérateur linéaire (6a, 6b) ; et/ou
dans lequel le système informatique (2) est configuré pour délivrer des signaux de commande à un ou plusieurs éléments d'équipements du système médical (1) et/ou au système d'entraînement (4) et/ou au dispositif de réalité augmentée (5), en particulier des signaux de commande pour inviter le ou les dispositifs d'imagerie à obtenir des données et/ou pour inviter le dispositif de réalité augmentée (5) à effectuer la superposition de l'indication de la direction de visée et/ou pour inviter le système d'entraînement (4) à entraîner un élément d'équipement (6a, 6b, 6c, 6d, 6e, 6f) ; et/ou
dans lequel le système médical (1) comprend un appareil de traitement par rayonnement comprenant une source de faisceau de traitement et une unité de support de patient, et dans lequel le système informatique (2) est couplé fonctionnellement à l'appareil de traitement par rayonnement pour délivrer un signal de commande à l'appareil de traitement par rayonnement afin de commander le fonctionnement de la source de faisceau de traitement et/ou la position de l'unité de support de patient.

16. Programme d'ordinateur qui, lorsqu'il est exécuté par un ordinateur ou lorsqu'il est chargé sur un ordinateur, amène l'ordinateur à exécuter les étapes de procédé du procédé selon l'une quelconque des revendications 1 à 13 ;
et/ou un support de stockage de programme sur lequel le programme informatique est stocké ;
et/ou un ordinateur comprenant au moins un processeur et une mémoire et/ou le support de stockage de programme, dans lequel le programme s'exécute sur l'ordinateur ou est chargé dans la mémoire de l'ordinateur ;
et/ou une onde de signal ou une onde de signal numérique, transportant des informations qui représentent le programme ;
et/ou un flux de données représentatif du programme.
